(19) Europäisches Patentamt
European Patent Office
Office européen des brevets

(11) EP 1 683 791 B1

(12) # EUROPÄISCHE PATENTSCHRIFT

(45) Veröffentlichungstag und Bekanntmachung des Hinweises auf die Patenterteilung:
**02.04.2008 Patentblatt 2008/14**

(51) Int Cl.:
***C07D 301/12*** *(2006.01)* ***C07D 303/04*** *(2006.01)*
***C07F 15/00*** *(2006.01)*

(21) Anmeldenummer: **06000267.2**

(22) Anmeldetag: **07.01.2006**

(54) **Verfahren zur Ruthenium-katalysierten Epoxidierung von Olefinen mit Wasserstoffperoxid**

Process for the epoxidation of olefins with hydrogen peroxide by ruthenium complex catalysed reaction

Procédé pour epoxydation de olefines catalysée par complexe de ruthenium avec du peroxyde d'hydrogène

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **20.01.2005 DE 102005002821**

(43) Veröffentlichungstag der Anmeldung:
**26.07.2006 Patentblatt 2006/30**

(73) Patentinhaber: **Saltigo GmbH**
**40764 Langenfeld (DE)**

(72) Erfinder:
- **Mägerlein, Wolfgang, Dr.**
  **50733 Köln (DE)**
- **Beller, Matthias, Prof. Dr.**
  **18211 Nienhagen (DE)**
- **Tse, Man-Kin, Dr.**
  **18055 Rostock (DE)**
- **Bhor, Santosh**
  **18059 Rostock (DE)**
- **Klawonn, Markus**
  **18106 Rostock (DE)**
- **Anilkumar, Gopinanthan**
  **18057 Rostock (DE)**

(74) Vertreter: **Wichmann, Birgid et al**
**LANXESS Deutschland GmbH**
**LIP-IPR**
**Q 18 / 1450**
**51369 Leverkusen (DE)**

(56) Entgegenhaltungen:
**EP-A- 1 561 750**

- **NISHIYAMA H ET AL: "novel ruthenium-pyridinedicarboxylate complexes of terpyridine and chiral bis(oxazolinyl)pyridine: a new catalytic system for alkene epoxidation with [bis(acetoxy) iodo]benzene as an oxygen donor" CHEMICAL COMMUNICATIONS - CHEMCOM, ROYAL SOCIETY OF CHEMISTRY, GB, 1997, Seiten 1863-1864, XP002331856 ISSN: 1359-7345**
- **TSE M K ET AL: "An improved protocol for the ruthenium(pybox)-catalyzed asymmetric alkene epoxidation" TETRAHEDRON LETTERS, ELSEVIER, AMSTERDAM, NL, Bd. 44, Nr. 40, 29. September 2003 (2003-09-29), Seiten 7479-7483, XP004453451 ISSN: 0040-4039**
- **STOOP RM ET AL: "ruthenium(II)complexes with chiral tetradentate P2N2 ligands catalyze the asymmetric epoxidation of olefins with H2O2" ORGANOMETALLICS, ACS, WASHINGTON, DC, US, Bd. 19, 2000, Seiten 4117-4126, XP002331847 ISSN: 0276-7333**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 683 791 B1

Printed by Jouve, 75001 PARIS (FR)

## Beschreibung

Die vorliegende Erfindung betrifft ein Verfahren zur Epoxidierung von Olefinen mit Katalysatoren auf Basis von Rutheniumkomplexen in Gegenwart von Wasserstoffperoxid.

Olefine sind gut verfügbare und preisgünstige Rohstoffe für industrielle Anwendungen. Eine besonders wichtige Reaktion für organische Synthesen stellt die Oxidation von Olefinen zu Epoxiden dar, welche vielseitige Zwischenstufen bei der Synthese von Wirkstoffen sowie Feinchemikalien (Kosmetik-, Polymerindustrie etc.) sind.

Außer molekularem Sauerstoff stellt Wasserstoffperoxid ein ökologisch nachhaltiges, "grünes" Oxidationsmittel dar, das zudem kostengünstig und breit verfügbar ist. In Epoxidierungsreaktionen werden mit Wasserstoffperoxid bis zu 47 % Atom-Effizienz erreicht und es entsteht lediglich Wasser als Nebenprodukt. Gegenüber Reaktionen mit reinem Sauerstoff (insbesondere Druckreaktionen mit Sauerstoff) besitzt Wasserstoffperoxid den Vorteil von weitaus geringerem Sicherheitsrisiko.

Traditionell lassen sich Epoxide aus Olefinen durch Reaktion mit Persäuren, welche durch die Einwirkung von Wasserstoffperoxid auf Säuren oder Säurederivate generiert werden können, herstellen. Ein Nachteil dieser Methode ist die Einschränkung der Substratbreite auf nicht-säureempfindliche Olefine und Epoxide sowie der Anfall von stöchiometrischen Mengen an Salzabfall. Zur Verbesserung dieser Nachteile und zur Erhöhung der Selektivität von Epoxidierungsreaktionen wurden Übergangsmetall-katalysierte Varianten mit Wasserstoffperoxid entwickelt. Hierbei ist der wahrscheinlich am breitesten einsetzbare Katalysator für die Olefin-Epoxidierung unter neutralen Bedingungen das MTO-System (Methyltrioxorhenium) [(a) Herrmann, W. A.; Fischer, R. W.; Marz, D. W. Angew. Chem. Int. Ed. 1991, 30, 1638-1641; (b) Rudolf, J.; Reddy, K. L.; Chiang, J. P.; Sharpless, K. B. J. Am. Chem. Soc. 1997, 119, 6189-6190. (c) Hermann, W. A.; Kratzer, R. M.; Ding, H.; Thiel, W. R.; Glas, H. J. Organomet. Chem. 1998, 555, 293-295.]. Jedoch ist aus technischer Sicht die Entwicklung eines preisgünstigeren und aktiveren sowie produktiveren Katalysatorsystems für chemoselektive Olefin-Epoxidierungen mit Wasserstoffperoxid ein wichtiges und anspruchsvolles Ziel.

Ein für Epoxidierungsreaktionen interessantes und preisgünstiges Edelmetall stellt Ruthenium dar. Ein Beispiel für eine Ruthenium-katalysierte Epoxidierung in Gegenwart von Wasserstoffperoxid findet sich in: Stoop, R. M.; Bachmann, S.; Valentini, M.; Mezzetti, A. Organornetallics 2000, 19, 4117-4126.). Hierin wird jedoch ausschließlich die Umsetzung von Styrolderivaten beschrieben und die Ausbeuten erreichen maximal 55 %. Ein weiteres katalytisches Epoxidierungssystem auf Basis von $RuCl_3$ und Pyridin-2,6-dicarbonsäure, das in Gegenwart von Wasserstoffperoxid verläuft, ist in Klawonn, M.; Tse, M. K.; Bhor, S.; Döbler, C.; Beller, M. J. Mol. Catal. A 2004, 218, 13-19 beschrieben. Nachteilig hierbei ist jedoch die Einschränkung der Olefin-Substratbreite auf nicht-säureempfindliche Olefine, da diese Reaktion unter sauren Bedingungen abläuft und somit die Toleranz von funktionellen Gruppen begrenzt. Ferner muss eine hohe Menge des Liganden (Pyridin-2,6-dicarbonsäure) zugesetzt werden, um eine ausreichende Produktausbeute zu erreichen.

Es besteht daher weiterhin das Bedürfnis, ein allgemeines chemoselektives, bei milden und gegebenenfalls pH-neutralen Bedingungen operierendes und gleichzeitig effizientes Verfahren zur Epoxidierung von Olefinen zu entwickeln. Ferner ist die Verwendung eines sowohl preisgünstigen als auch umweltfreundlichen Oxidationsmittels ein aus technischer Sicht anzustrebendes Ziel.

Es wurde nun gefunden, dass Ruthenium-Katalysatorsysteme, die mit Terpyridin-Liganden und mit 2,6-Pyridindicarbonsäure-Liganden modifiziert sind, die Umsetzung von Olefinen zu Epoxiden in Gegenwart von Wasserstoffperoxid unter milden, unter anderem auch pH-neutralen Bedingungen effizient und mit hohen Produktivitäten bewirken.

Das gefundene Verfahren bezieht sich auf die Herstellung von Epoxiden der Formel (I),

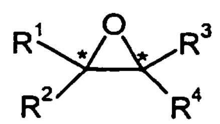

(I)

in der

$R^1$, $R^2$, $R^3$ und $R^4$ jeweils unabhängig voneinander für Wasserstoff, Alkyl, Aryl, Arylalkyl, Halogenalkyl oder Reste der Formel (IIa) bis (IIf) stehen

| | | | |
|---|---|---|---|
| A-B-D-E | (IIa) | A-E | (IIb) |
| $A\text{-}SO_2\text{-}E$ | (IIc) | $A\text{-}B\text{-}SO_2R^6$ | (IId) |
| $A\text{-}SO_3W$ | (IIe) | A-COW | (IIf) |

wobei in den Formeln (IIa) bis (IIf)

A fehlt oder für einen Alkylen- oder Halogenalkylenrest steht und

B fehlt oder für Sauerstoff oder $NR^5$ steht, wobei

$R^5$ für Wasserstoff, Arylalkyl oder Aryl steht und

D für eine Carbonyl-Gruppe steht und

E für $R^6$, $OR^6$, $NHR^7$ oder $N(R^7)_2$ steht,wobei

$R^6$ für Alkyl, Arylalkyl oder Aryl und

$R^7$ jeweils unabhängig für Alkyl, Arylalkyl oder Aryl steht oder $N(R^7)_2$ zusammen für einen cyclischen Aminorest mit 4 bis 12 Kohlenstoffatomen steht und

W für OH, $NH_2$, oder OM steht, wobei M ein Alkalimetallion, ein halbes Äquivalent eines Erdalkalimetallions, ein Ammoniumion oder ein organisches Ammoniumion steht

oder jeweils zwei der Reste $R^1$, $R^2$, $R^3$ und $R^4$ zusammen Teil eines 3 bis 7-gliedrigen Cyclus sind, der insgesamt 3 bis 16 Kohlenstoffatome umfasst,
das dadurch gekennzeichnet ist, dass Verbindungen der Formel (III),

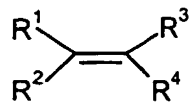

(III)

in der $R^1$, $R^2$, $R^3$ und $R^4$ jeweils unabhängig voneinander die vorstehend genannte Bedeutung besitzen,
mit Wasserstoffperoxid ($H_2O_2$) umgesetzt werden,
wobei die Umsetzung in Gegenwart eines Rutheniumkomplexes erfolgt, der als Liganden sowohl Verbindungen der Formel (IV) trägt

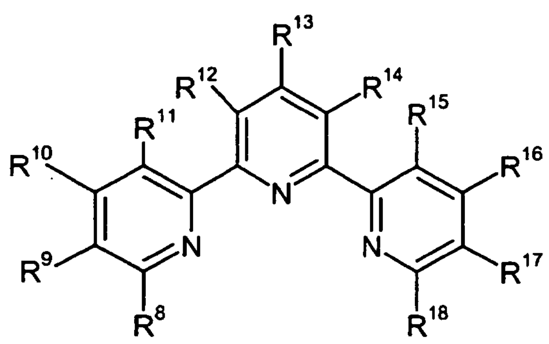

(IV)

,

in der $R^8$, $R^9$, $R^{10}$, $R^{11}$, $R^{12}$, $R^{13}$, $R^{14}$, $R^{15}$, $R^{16}$, $R^{17}$ und $R^{18}$ jeweils unabhängig voneinander für Wasserstoff, Halogen, Hydroxy, Hydroxycarbonyl, Alkoxycarbonyl, Alkoxy, Alkyl, Arylalkyl oder Aryl stehen, oder
jeweils zwei der Reste $R^8$, $R^9$, $R^{10}$ und $R^{11}$ oder jeweils zwei der Reste $R^{15}$, $R^{16}$, $R^{17}$ und $R^{18}$ zusammen Teil eines 3 bis 7-gliedrigen Monocyclus sind, der insgesamt 3 bis 16 Kohlenstoffatome umfasst, oder zusammen Teil eines Bicyclus sind, der insgesamt 3 bis 16 Kohlenstoffatome umfasst,
als auch Verbindungen der Formel (V) trägt

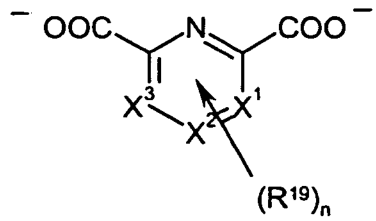

(V)

in der

| | |
|---|---|
| $X^1$, $X^2$ und $X^3$ | jeweils unabhängig voneinander für N, CH bzw. $CR^{19}$ stehen und |
| $R^{19}$ | für Wasserstoff, Halogen, Hydroxy, Hydroxycarbonyl, Alkoxycarbonyl, Alkoxy, Alkoxyalkyl, Arylalkyl oder Aryl steht und |
| n | für 0, 1, 2 oder 3, bevorzugt für 0 oder 1 und besonders bevorzugt für 0 steht. |

Der Rahmen der Erfindung umfasst alle oben stehenden und im Folgenden aufgeführten, allgemeinen oder in Vorzugsbereichen genannten Restedefinitionen, Parameter und Erläuterungen in beliebiger Kombination untereinander, also auch zwischen den jeweiligen Bereichen und Vorzugsbereichen.

Aryl steht im Rahmen der Erfindung sofern nicht gesondert vermerkt bevorzugt für carbocyclische aromatische Reste mit 6 bis 24 Gerüstkohlenstoffatomen oder heteroaromatische Reste mit 5 bis 24 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können. Weiterhin können die carbocyclischen aromatischen Reste oder heteroaromatische Reste mit bis zu fünf gleichen oder verschiedenen Substituenten pro Cyclus substituiert sein, ausgewählt aus der Gruppe Hydroxy, Halogen, Nitro, Cyano, freies oder geschütztes Formyl, $C_1$-$C_{12}$-Alkyl, $C_1$-$C_{12}$-Halogenalkyl, $C_5$-$C_{14}$-Aryl, $C_6$-$C_{15}$-Arylalkyl, $C_1$-$C_{12}$-Alkoxy, $C_1$-$C_{12}$-Alkoxycarbonyl, -PO-[(C,-$C_8$)-Alkyl]$_2$, -PO-[($C_5$-$C_{14}$)-Aryl]$_2$, -PO-[($C_1$-$C_8$)-Alkyl)($C_5$-$C_{14}$)-Aryl)], Tri($C_1$-$C_8$-alkyl)siloxyl oder Resten der Formeln (IIa) bis (IIf). Gleiches gilt für den Arylteil eines Arylalkyl-Restes.

Beispielsweise steht Aryl besonders bevorzugt für Phenyl, Naphthyl oder Anthracenyl, das gegebenenfalls einfach, zweifach oder dreifach durch Reste substituiert ist, die jeweils unabhängig voneinander ausgewählt sind aus der Gruppe $C_1$-$C_6$-Alkyl, $C_1$-$C_6$-Halogenalkyl, $C_5$-$C_{14}$-Aryl, $C_{1-}C_6$-Alkoxy, $C_1$-$C_6$-Alkoxycarbonyl, Halogen, Hydroxy, Nitro oder Cyano.

**Alkyl** bzw. **Alkylen** bzw. **Alkoxy** steht im Rahmen der Erfindung sofern nicht gesondert vermerkt bevorzugt jeweils unabhängig für einen gegebenenfalls substituierten geradkettigen, cyclischen, verzweigten oder unverzweigten Alkyl- bzw. Alkylen- bzw. Alkoxy-Rest,. Gleiches gilt für den Alkylenteil eines Arylalkyl-Restes. Als Substituenten für die Alkyl- bzw. Alkylen- bzw. Alkoxyreste kommen beispielweise $C_1$-$C_6$-Alkyl, $C_1$-$C_6$-Halogenalkyl, $C_5$-$C_{14}$-Aryl, $C_6$-$C_{15}$-Arylalkyl, $C_1$-$C_6$-Alkoxy, $C_1$-$C_6$-Aryloxy, $C_1$-$C_6$-Alkoxycarbonyl, $C_1$-$C_6$-Acyloxy, Halogen, Hydroxy, Nitro, Cyano oder Tri ($C_1$-$C_8$-alkyl)siloxyl in Frage.

Beispielsweise steht Alkyl besonders bevorzugt für Methyl, Ethyl, n-Propyl, Isopropyl, n-Butyl, tert.-Butyl, n-Pentyl, Cyclohexyl und n-Hexyl, n-Heptyl, n-Octyl, iso-Octyl, n-Decyl und n-Dodecyl.

Beispielsweise steht Alkylen bevorzugt für Methylen, 1,1-Ethylen, 1,2-Ethylen, 1,1-Propylen, 1,2-Propylen, 1,3-Propylen, 1,1-Butylen, 1,2-Butylen, 2,3-Butylen und 1,4-Butylen, 1,5-Pentylen, 1,6-Hexylen, 1,1-Cyclohexylen, 1,4-Cyclohexylen, 1,2-Cyclohexylen und 1,8-Octylen.

Beispielsweise steht Alkoxy bevorzugt für Methoxy, Ethoxy, Isopropoxy, n-Propoxy, n-Butoxy, tert.-Butoxy und Cyclohexyloxy.

Cyclische Alkylreste können sowohl 3 bis 7-gliedrige Homo- als auch Heterocyclen mit insgesamt 3 bis 17 Kohlenstoffatomen, letztere vorzugsweise mit 1, 2 oder 3 Heteroatomen, sein. Homocyclische Alkylreste sind beispielsweise gegebenenfalls substituiertes Cyclopentyl oder Cyclohexyl, Beispiele für heterocyclische Alkylreste sind Dioxolan- oder Phthalimidreste.

In Tri($C_1$-$C_8$-alkyl)siloxyl-Substituenten können dabei die $C_1$-$C_8$-Alkyl-Reste gleich oder verschieden sein. Ein Beispiel für einen solchen Substituenten ist tert-Butyldimethylsiloxyl.

**Arylalkyl** steht im Rahmen der Erfindung sofern nicht gesondert vermerkt bevorzugt jeweils unabhängig für einen geradkettigen, cyclischen, verzweigten oder unverzweigten Alkylrest, der einfach oder mehrfach, besonders bevorzugt einfach durch Arylreste gemäß vorstehender Definition substituiert ist.

**Halogenalkyl** bzw. **Halogenalkylen** steht im Rahmen der Erfindung sofern nicht gesondert vermerkt bevorzugt

jeweils unabhängig für einen geradkettigen, cyclischen, verzweigten oder unverzweigten Alkylrest, der mit einem, mehreren oder vollständig mit Halogenatomen unabhängig voneinander ausgewählt aus der Gruppe Fluor, Chlor, Brom und Iod substituiert sein kann.

Beispielsweise steht $C_1$-$C_8$-Halogenalkyl besonders bevorzugt für Trifluormethyl, Trichlormethyl, 2,2,2-Trifluorethyl, Pentafluorethyl und Nonafluorbutyl.

Halogen kann für Fluor, Chlor, Brom oder Iod, bevorzugt für Fluor oder Chlor stehen.

**Geschütztes Formyl** bedeutet einen Formyl-Rest, der durch Überführung in ein Aminal, Acetal oder ein gemischtes Aminalacetal geschützt ist, wobei die Aminale, Acetale und gemischten Aminalacetale acyclisch oder cyclisch sein können.

Die mit * gekennzeichnete Kohlenstoffatom in der allgemeinen Formel (I) können je nach Bedeutung von $R^1$ bis $R^4$ unabhängig voneinander asymmetrische Kohlenstoffatome sein, die unabhängig voneinander *(R)-* oder *(S)-*Konfiguration aufweisen können. Im Rahmen der Erfindung können beide, eines der beiden oder keines der beiden mit * gekennzeichneten Kohlenstoffatome in der allgemeinen Formel (I) asymmetrisch sein.

Im Folgenden werden bevorzugte Verbindungen der Formeln (I), (IV) und (V) definiert.

In Formel (I) stehen bevorzugt $R^1$, $R^2$, $R^3$ und $R^4$ jeweils unabhängig voneinander für Wasserstoff, gegebenenfalls substituiertes $C_1$-$C_8$-Alkyl, $C_5$-$C_{14}$-Aryl, $C_6$-$C_{15}$-Arylalkyl, $C_1$-$C_8$-Halogenalkyl oder es sind jeweils zwei der Reste $R^1$, $R^2$, $R^3$ und $R^4$ zusammen Teil eines 3 bis 7-gliedrigen Cyclus, der insgesamt 3 bis 16 Kohlenstoffatome umfasst.

Besonders bevorzugt sind dabei Verbindungen der Formel (I), in denen mindestens ein Rest aus $R^1$, $R^2$, $R^3$ und $R^4$ für gegebenenfalls substituiertes $C_5$-$C_{14}$-Aryl steht oder jeweils zwei der Reste $R^1$, $R^2$, $R^3$ und $R^4$ zusammen Teil eines 3 bis 7-gliedrigen Cyclus sind, der insgesamt 3 bis 16 Kohlenstoffatome umfasst.

In Formel (IV) stehen bevorzugt $R^8$, $R^9$, $R^{11}$, $R^{12}$, $R^{14}$, $R^{15}$, $R^7$ sowie $R^{18}$ für Wasserstoff und gleichzeitig $R^{10}$, $R^{13}$ sowie $R^{16}$ für tert-Butyl, $R^8$, $R^9$, $R^{10}$, $R^{11}$, $R^{12}$, $R^{13}$, $R^{14}$, $R^{15}$, $R^{16}$, $R^{17}$ und $R^{18}$ für Wasserstoff oder jeweils zwei der Reste $R^8$, $R^9$, $R^{10}$ und $R^{11}$ und jeweils zwei der Reste $R^{15}$, $R^{16}$, $R^{17}$ und $R^{18}$ sind zusammen Teil eines 3 bis 7-gliedrigen Monocyclus, der insgesamt 3 bis 16 Kohlenstoffatome umfasst, oder zusammen Teil eines Bicyclus, der insgesamt' 3 bis 16 Kohlenstoffatome umfasst, und die übrigen Reste aus $R^8$, $R^9$, $R^{10}$, $R^{11}$, $R^{12}$, $R^{13}$, $R^{14}$, $R^{15}$, $R^{16}$, $R^{17}$ und $R^{18}$ stehen für Wasserstoff. In einer bevorzugten Ausführungsform stehen $R^8$, $R^9$, $R^{10}$, $R^{11}$, $R^{12}$, $R^{13}$, $R^{14}$, $R^{15}$, $R^{16}$, $R^{17}$ und $R^{18}$ für Wasserstoff.

In Verbindungen der Formel (V) stehen von den Resten $X^1$, $X^2$, $X^3$ bevorzugt mindestens zwei, besonders bevorzugt drei für CH bzw $CR^{19}$ und ganz besonders bevorzugt für CH. n steht bevorzugt für 0 oder besonders bevorzugt für 0 oder 1 mit einem Substituenten in 4-Stellung und ganz besonders bevorzugt für 0.

Bevorzugte Rutheniumkomplexe sind solche der Formel (VI)

[Ru(IV)(V)] (VI)

in der (IV) für eine Verbindung der Formel (IV) und (V) für eine Verbindung der Formel (V) steht. Solche Komplexe können in an sich bekannter Weise analog zur eingangs zitierten Literatur hergestellt werden (Nishiyama, H.; Shimada, T.; Itoh, H.; Sugiyama, H.; Motoyama, Y. Chem. Commun. 1997, 1863-1864).

Das erfindungsgemäße Verfahren wird in einer bevorzugten Ausführungsform in Gegenwart von organischem Lösungsmittel wie insbesondere sekundären oder tertiären Alkoholen, aprotisch polaren Lösungsmitteln, Ketonen, chlorierten Kohlenwasserstoffen und aromatischen Kohlenwasserstoffen durchgeführt. Unter aprotischen polaren Lösungsmittel sind solche zu verstehen, die bei 25°C eine Dielektrizitätskonstante von 5 oder mehr und einen pKs-Wert bezogen auf eine wässrige Bezugsskala bei 25°C von 20 oder mehr aufweisen. Für das erfindungsgemäße Verfahren besonders bevorzugt sind sekundäre und tertiäre Alkohole wie insbesondere t-Amylalkohol und t-Butylalkohol.

Die Reaktion wird beispielsweise so durchgeführt, dass die Verbindungen der Formel (III) und der Rutheniumkomplex in einem organischen Lösungsmittel vorgelegt und mit dem Oxidationsmittel, welches gegebenenfalls in einem geeigneten organischen Lösungsmittel gelöst ist, versetzt werden. In einer bevorzugten Ausführungsform wird eine Lösung des Oxidationsmittels über einen Zeitraum von 10 Minuten bis 24 Stunden zum Reaktionsgemisch zudosiert.

Eine etwaige zusätzliche Nachrührzeit kann beispielsweise bis zu 24 Stunden, bevorzugt bis zu 5 Stunden und besonders bevorzugt bis zu 1 Stunde betragen.

Die Reaktion kann bei Temperaturen von -20°C bis 150°C durchgeführt werden, bevorzugt bei 0 bis 80°C, besonders bevorzugt bei 0°C bis 40°C und ganz besonders bevorzugt bei 15°C bis 30°C.

Der Druck bei der Reaktion ist unkritisch und kann beispielsweise 0,5 bis 100 bar, bevorzugt 0,8 bis 10 bar betragen. Besonders bevorzugt ist Umgebungsdruck.

Das Oxidationsmittel Wasserstoffperoxid wird bevorzugt in einer Menge von 1 bis 10 Moläquivalenten bezogen auf Verbindungen der Formel (III), besonders bevorzugt von 1 bis 5 Moläquivalenten und ganz besonders bevorzugt von 1 bis 3 Moläquivalenten eingesetzt. Das Oxidationsmittel wird gegebenenfalls vorteilhaft als Lösung in einem Lösungsmittel eingesetzt, besonders bevorzugt als Lösung in Wasser und gegebenenfalls zusätzlich wenigstens einem der vorangehend aufgeführten organischen Lösungsmittel.

Im Rahmen der Erfindung kann der Rutheniumkomplex entweder als isolierter Komplex eingesetzt werden oder in situ in der Reaktionsmischung erzeugt werden. In letzterem Falle werden eine geeigneter Rutheniumprecursor, wie z.B. $[Ru(p\text{-cymol})Cl_2]_2$, und die beiden Liganden der Formeln (IV) und (V) in der Reaktionsmischung zusammengegeben.

Die Herstellung der isolierten Komplexe erfolgt vorzugsweise ebenfalls durch Zusammengeben eines geeigneten Rutheniumprecursors, wie z.B. $[Ru(p\text{-cymol})Cl_2]_2$, und der beiden Liganden der Formeln (IV) und (V) in der Weise, dass beispielsweise der Rutheniumprecursor mit dem Liganden der Formel (IV) in einem geeigneten Lösungsmittel in Inertgasatmosphäre vorgelegt und eine Lösung des Liganden der Formel (V), vorzugsweise in Form seines Di-Natriumsalzes, zugegeben wird, die Reaktionsmischung anschließend erhitzt und der Rutheniumkomplex beispielsweise durch Kristallisation, Filtration und Umkristallisation isoliert wird. Insbesondere bei Verwendung isolierter Komplexe als Katalysatoren kann das erfindungsgemäße Verfahren bei pH-neutralen Bedingungen durchgeführt werden, was eine größere Anwendungsbreite hinsichtlich der Verbindungen der Formel (III) eröffnet. Daher ist der Einsatz isolierter Komplexe der Formel (IV) bevorzugt.

Als Rutheniumprecursor eignen sich beispielsweise Rutheniumverbindungen wie Ru(III)-chlorid oder beispielsweise Ru(II)- oder Ru(III)-Komplexe, die wenigstens einen Liganden aus der Gruppe Phosphane, wie z.B. Triarylphosphane, Trialkylphosphane oder Bis(diarylphosphino)alkane, Amine, wie z.B. Triarylamine, Trialkylamine, cycloaliphatische oder cycloaromatische bzw. heteroaromatische Amine, oder ungesättigte cyclische Kohlenwasserstoffe, wie z.B. p-Cymol, Norbornadien oder Cyclooctadien. Ein Beispiel für einen geeigneten Rutheniumprecursor ist $[Ru(p\text{-cymol})Cl_2]_2$.

Die Reaktion kann unter pH-neutralen Bedingungen durchgeführt werden, gegebenenfalls kann auch der Zusatz von Säuren oder Basen vorteilhaft sein. Bevorzugt wird die Reaktion unter pH-neutralen Bedingungen durchgeführt, wobei hierbei pH-Werte von 5 bis 9, gemessen bei 20 °C, zu verstehen sind.

Die Menge des eingesetzten Rutheniumkomplexes oder des eingesetzten Rutheniumprecursors liegt im Rahmen der Erfindung beispielsweise zwischen 0.001 und 20 mol%, bevorzugt zwischen 0.01 und 1 mol% und besonders bevorzugt zwischen 0.1 und 1 mol%.

Auf erfindungsgemäße Weise können Verbindungen der Formel (I) in sehr guten Ausbeuten unter milden Bedingungen erhalten werden. Die Aufarbeitung kann in an sich bekannter Weise z.B. durch Quenchen mit Wasser, Extraktion mit einem geeigneten organischen Lösungsmittel und Destillation oder Umkristallisation des Epoxids erfolgen.

Das erfindungsgemäße Verfahren kann sowohl stereoselektiv als auch nicht stereoselektiv durchgeführt werden. Bevorzugt wird das erfindungsgemäße Verfahren nicht stereoselektiv durchgeführt. Von nicht stereoselektiver Durchführung des erfindungsgemäßen Verfahrens ist im Rahmen der Erfindung auch eine racemische Durchführung umfasst. Es kann jedoch auch bevorzugt sein, das erfindungsgemäße Verfahren stereoselektiv durchzuführen.

Für eine solche stereoselektive (enantioselektive) Epoxidierung eignen sich bevorzugt solche Katalysatoren der Formel (VI), worin in den Verbindungen der Formel (IV) jeweils zwei der Reste $R^8$, $R^9$, $R^{10}$ und $R^{11}$ oder jeweils zwei der Reste $R^{15}$, $R^{16}$, $R^{17}$ und $R^{18}$ zusammen Teil eines 3 bis 7-gliedrigen Bicyclus sind, der insgesamt 3 bis 16 Kohlenstoffatome umfasst, wobei sich dieser Bicyclus besonders bevorzugt von einem Terpen ableitet.

Beispiele für solche Katalysatoren der Formel (VI) sind die Verbindungen der Formeln (VI-2) bis (VI-5),

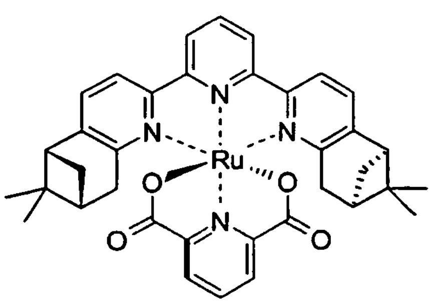

(VI-2)

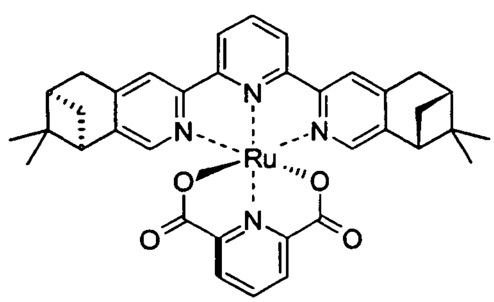

(VI-3)

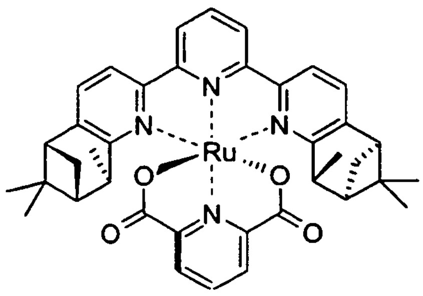
(VI-4)

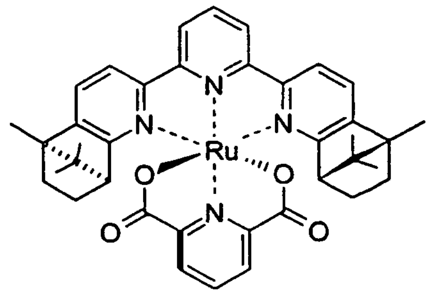
(VI-5)

die bisher in der Literatur nicht beschrieben und somit ebenfalls Gegenstand der vorliegenden Erfindung sind.

Bei der enantioselektiven Durchführung des erfindungsgemäßen Verfahrens wird eines der jeweiligen beiden Enantiomeren der allgemeinen Formel (I), in einem Enantiomerenüberschuss, im Folgenden auch ee ("enantiomeric excess") genannt, im Vergleich zum anderen Enantiomer erhalten. Bevorzugt beträgt dieser Enantiomerenüberschuss 2 bis 100 % ee, besonders bevorzugt 50 % bis 100 %. Eine Definition des ee-Werts wird im Rahmen der Beispiele dieser Anmeldung angegeben. Für den Fall, dass beide mit * gekennzeichneten Kohlenstoffatome in der allgemeinen Formel (I) asymmetrisch sind, kann es sich bei den beiden Enantiomeren auch um ein Diastereomerenpaar handeln.

Die erfindungsgemäß herstellbaren Verbindungen der Formel (I) eignen sich insbesondere zur Herstellung von Arzneimitteln, Agrochemikalien, Polymeren oder Zwischenprodukten davon.

Das erfindungsgemäße Verfahren zeichnet sich dadurch aus, dass die Epoxidierung von Olefinen unter sehr milden Bedingungen bei hoher Chemoselektivität verläuft und sehr gute Produktausbeuten liefert. Besonders zu erwähnen sind die sehr geringen benötigten Mengen an Ruthenium und an Liganden. Gleichzeitig ist die Möglichkeit der Verwendung des preisgünstigen Oxidationsmittels Wasserstoffperoxid ein besonderer Vorteil und weiterhin die Möglichkeit, unter pH-neutralen Bedingungen auch säureempfindliche Verbindungen der Formel (III) umzusetzen und/oder säureempfindliche Verbindungen der Formel (I) herzustellen.

**Beispiele**

**Allgemeine Arbeitsvorschrift:**

In einem typischen Experiment wird der Rutheniumkomplex [Ru(2,2':6',2"terpyridin)(pyridin-2,6-dicarboxylat)] (VI-1) (0.0025 mmol) in tert-Amylalkohol (9 ml) bei Raumtemperatur gerührt und das Olefin der Formel (III) (0.5 mmol) zugesetzt. Zu dieser Mischung wird eine Lösung von 30 %igem Wasserstoffperoxid (1.5 mmol) in t-Amylalkohol (0.83 ml) innerhalb von 12 h dosiert. Danach wird die Reaktion durch Zusatz von Wasser (10 ml) und $Na_2SO_3$ (0-5 g) gequencht und mit Ethylacetat (20 ml) extrahiert. Nach Trocknung der organischen Phase werden Aliquote mittels Gaschromatographie analysiert. Zur Isolierung der Epoxide wird das Produkt nach destillativer Entfernung des Solvens ggf. durch Säulenchromatographie gereinigt.

**Beispiele 1 - 25:**

Die Tab. 1 fasst die Beispiele zur Epoxidierung von Olefinen der Formel (III) gemäß der allgemeinen Arbeitsvorschrift in Gegenwart des Rutheniumkomplexes [Ru(2,2':6',2"terpyridin)-(pyridin-2,6-dicarboxylat)] (VI-1) zusammen:

**Tab.1:** Epoxidierung von Olefinen der Formel (III) gemäß der allgemeinen Arbeitsvorschrift in Gegenwart des Rutheniumkomplexes [Ru(2,2':6',2''terpyridin)-(pyridin-2,6-dicarboxylat)] (VI-1)

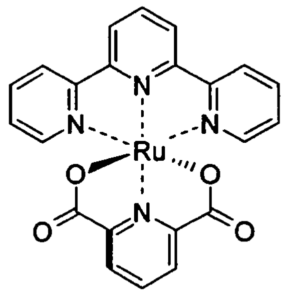

(VI-1)

| Bsp. | Olefin der Formel (III) | Umsatz (%) | Ausbeute (%) |
|---|---|---|---|
| 1 | 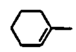 | 100 | 84 |
| 2 | 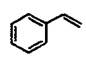 | 100 | 71 |
| 3 | 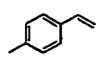 | 100 | 83 |
| 4 | 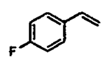 | 100 | 86 |
| 5 | 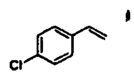 | 100 | >99 |
| 6 | 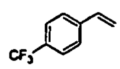 | 90 | 89 |
| 7 | 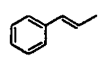 | 100 | >99 |
| 8 | 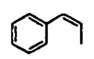 | 100 | >99 |
| 9 | 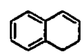 | 100 | 80 |
| 10 | 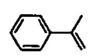 | 100 | 88 |
| 11 | 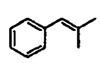 | 100 | >99 |
| 12 | 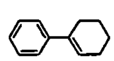 | 100 | 91 |
| 13 | 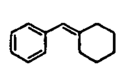 | 100 | 86 |
| 14 | 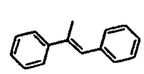 | 100 | 95 |

(fortgesetzt)

| Bsp. | Olefin der Formel (III) | Umsatz (%) | Ausbeute (%) |
|---|---|---|---|
| 15 | 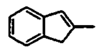 | 100 | 86 |
| 16 | 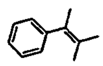 | 100 | 96 |
| 17 | 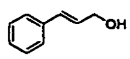 | 100 | 97 |
| 18[(a)] | 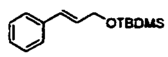 | 100 | >99 |
| 19[(b)] | 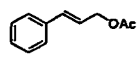 | 100 | 98 |
| 20 | 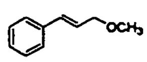 | 100 | 96 |
| 21 | 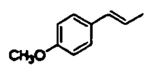 | 100 | 99 |
| 22 | 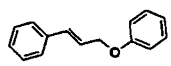 | 100 | 92 |
| 23 | 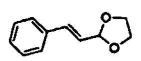 | 100 | 62 |
| 24 | 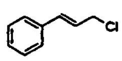 | 100 | 94 |
| 25 | 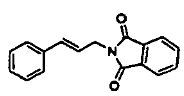 | > 90 | 81 |

(a) OTBDME = *tert*-Butyldimethylsiloxyl;
(b) Ac = Acetyl

Im Folgenden sind die Analysendaten der in den Beispielen hergestellten Epoxide der Formel (I) aufgeführt.

**1,2-Epoxy-1-methylcyclohexan:** $^1H$ NMR (400.1 MHz, $CDCl_3$): δ 1.22 (m, 5 H), 1.36-1.31 (m, 2 H), 1.59 (m, 2 H), 1.82-1.78 (m, 2 H), 2.87 (s, 1 H); $^{13}C$ NMR (100.6 MHz, $CDCl_3$, ppm): δ 19.7, 20.1, 22.7, 25.0, 29.9, 57.8, 59.6; (E.I., 70 eV): *m/z* 112 ($M^+$), 111, 97 (100), 55, 43.

**Phenyloxiran:** $^1H$ NMR (400.1 MHz, $CDCl_3$): δ 2.72 (dd, *J* =5.6, 2.6 Hz, 1 H), 3.06 (dd, *J* = 5.6, 4.2 Hz, 1 H), 3.78 (dd, *J* = 4.2, 2.6 Hz, 1 H), 7.16 - 7.29 (m, 5 H); $^{13}C$ NMR (100.6 MHz, $CDCl_3$): δ 51.3, 52.5, 125.6, 128.3, 128.6, 137.7; (E.I., 70 eV): *m/z* 120 ($M^+$, 41), 119 (65), 92 (37), 91 (100), 90 (64), 89 (79).

**2-(p-Tolyl)-oxiran**: $^1H$ NMR (400.1 MHz, $CD_2Cl_2$): δ 2.33 (s, 3 H), 2.77 (dd, *J* = 5.5, 2.6 Hz, 1 H), 3.09, (dd, *J* = 5.5, 4.1 Hz, 1 H), 3.79, (dd, *J* = 4.1, 2.6 Hz, 1 H), 7.06-7.26 (m, 5 H); C NMR (100.6 MHz, $CD_2Cl_2$): δ 20.9, 50.9, 52.1, 125.5, 129.2, 134.8, 138.1; GC-MS: *m/z* 134 ($M^+$).

**4-Fluorphenyloxiran:** $^1H$ NMR (400.1 MHz, $CDCl_3$): δ 2.67 (dd, *J* = 5.6, 2.6 Hz, 1 H), 3.04 (dd, *J* = 5.6, 4.0 Hz, 1 H), 3.75 (dd, *J* = 4.0, 2.6 Hz, 1 H), 6.91- 6.96 (m, 2 H), 7.12 - 7.17 (m, 2H), $^{13}C$ NMR (100.6 MHz, $CDCl_3$): δ 51.6, 52.2, 115.9 (d, *J* = 20 Hz), 127.6 (d, *J* = 7 Hz), 133.7 (d, *J* = 2 Hz), 163.1 (d, *J* = 24 Hz); (E.I., 70 eV): *m/z* 138 ($M^+$), 137 ($M-1^+$), 122 (86), 109 (100), 96.

**4-Chlorphenyloxiran:** $^1H$ NMR (400.1 MHz, $CDCl_3$): δ 2.68 (dd, *J* = 5.6, 2.6 Hz, 1 H), 3.07 (dd, *J* = 5.6, 4.0 Hz, 1 H), 3.76 (dd, *J* = 4.0, 2.6 Hz, 1 H), 7.12-7.26 (m, 4 H); C NMR (100.6 MHz, $CDCl_3$): δ 51.4, 51.9, 127.0, 128.8, 134.1, 136.3; (E.I., 70 eV): *m/z* 156 ($M+2^+$, 9), 155 ($M+1^+$, 10), 154 ($M^+$, 28), 153 ($M-1^+$, 23), 125 (53), 119 (74), 89 (106).

**(4-Trifluormethyl)phenyloxiran:** $^1H$ NMR (400.1 MHz, $CDCl_3$): δ 2.77 (dd, *J* = 5.6, 2.6 Hz, 1 H), 3.19 (dd, *J*

= 5.6, 4.0 Hz, 1 H), 3.92 (dd, *J* = 4.0, 2.6 Hz, 1 H), 7.4 (d, *J* = 8.1 Hz, 2 H), 7.6 (d, *J* = 8.1 Hz, 2 H); $^{13}C$ NMR (100.6 MHz, $CDCl_3$): δ 51.4, 51.6, 125.4 (q, *J* = 3.8 Hz), 125.9, 141.9; (E.I., 70 eV): *m*/*z* 188 ($M^+$, 14), 187 (20), 159 (49), 158 (48), 119 (100), 91 (37).

***Trans*-2-Methyl-3-phenyloxiran:** $^1H$ NMR (400.1 MHz, $CDCl_3$): δ 1.44 (d, *J* = 5.2 Hz, 3 H), 3.03 (dq, *J* = 5.2, 2.0 Hz, 1 H), 3.57 (d, *J* = 2.0 Hz, 1 H), 7.23-7.4 (m, 5 H); $^{13}C$ NMR (100.6 MHz, $CDCl_3$): δ 18.0, 59.2, 59.6, 125.7, 128.1, 128.5, 137.9; (E.I., 70 eV): *m*/*z* 134 ($M^+$, 52), 133 (65), 105 (51), 91 (42), 90 (100), 89 (77), 77 (23).

***Cis*-2-Methyl-3-phenyloxiran:** $^1H$ NMR (400.1 MHz, $CDCl_3$): δ 1.07 (d, *J* = 5.4 Hz, 3 H), 3.33 (dd, *J* = 5.4, 4.3 Hz, 1 H), 4.05 (d, *J* = 4.3 Hz, 1 H), 7.25-7.36 (m, 5 H); $^{13}C$ NMR (100.6 MHz, $CDCl_3$): δ 12.5, 55.1, 57.5, 126.5, 127.4, 128.0, 135.5; MS (E.I., 70 eV): *m*/*z* 134 ($M^+$).

**1,2-Dihydronaphthalinoxid:** $^1H$ NMR (400.1 MHz, $CDCl_3$): δ 1.72-1.80 (m, 1 H), 2.41 (dddd, *J* = 14.5, 6.5, 2.9, 1.7 Hz, 1 H), 2.55 (dd, *J* = 15.5, 5.6 Hz, 1 H), 2.76-2.85 (m, 1 H), 3.72-3.74 (m, 1 H), 3.85 (d, *J* = 4.4 Hz, 1 H), 7.10 (d, *J* = 7.3 Hz, 1 H), 7.19-7.23 (m, 1 H), 7.25-7.29 (m, 1 H), 7.40 (dd, *J* = 7.3, 1.4 Hz, 1 H); $^{13}C$ NMR (100.6 MHz, $CDCl_3$): δ 21.7, 24.3, 52.6, 54.9, 126.0, 128.2, 128.3, 129.4, 132.4, 136.5; GC-MS: *m*/*z* 146 ($M^+$).

**2-Methyl-2-phenyloxiran:** $^1H$ NMR (400.1 MHz, $CDCl_3$): δ 1.65 (s, 3 H), 2.73 (d, *J* = 5.4 Hz, 1 H), 2.90 (d, *J* = 5.4 Hz, 1 H), 7.17 - 7.31 (m, 5 H), $^{13}C$ NMR (100.6 MHz, $CDCl_3$): δ 56.9, 57.2, 125.4, 127.6, 128.5, 141.3; MS (E.I., 70 eV): *m*/*z* 134 ($[M]^+$, 35), 133 (87), 105 (100), 104 (41), 103 (58), 91 (23), 79 (37), 78 (54), 77 (49).

**2,2-Dimethyl-3-phenyloxiran:** $^1H$ NMR (400.1 MHz, $CDCl_3$): δ 1.04 (s, 3 H), 1.45 (s, 3 H), 3.83 (s, 1 H), 7.21-7.33 (m, 5 H); $^{13}C$ NMR (100.6 MHz, $CDCl_3$): δ 17.9, 24.7, 61.0, 64.5, 126.3, 127.3, 128, 136.6; MS (E.I, 70 eV) *m*/*z* 148 ($M^+$).

**1,2-Epoxy-1-phenyl-cyclohexan:** $^1H$ NMR (400.1 MHz, $CDCl_3$): δ 1.18 - 1.30 (m, 1 H), 1.34 - 1.44 (m, 1H), 1.44 - 1.58 (m, 2 H), 1.87 - 1.95 (m, 2 H), 2 - 2.09 (m, 1 H), 2.16 - 2.25 (m, 1 H), 2.99 (m, 1 H), 7.15 - 7.20 (m, 1 H), 7.23 - 7.32 (m, 4 H); $^{13}C$ NMR (100.6 MHz, $CDCl_3$): δ 19.9, 20.2, 24.8, 29.0, 60.3, 62.1, 125.4, 127.3, 128.4, 142.6, MS (E.I., 70 eV): *m*/z = 175 ($[M+1]^+$, 10), 174 ($[M]^+$, 82), 173 (100), 159 (21), 145 (40), 129 (50), 117 (47), 115 (58), 105 (68), 91 (58), 77 (43).

**2-Phenyl-1-oxaspiro[2.5]octan:** $^1H$ NMR (400.1 MHz, $CDCl_3$): δ 1.22-1.31 (m, 2 H), 1.37-1.85 (m, 8 H), 3.85 (s, 1 H), 7.23-7.34 (m, 5 H); $^{13}C$ NMR (100.6 MHz, $CDCl_3$): δ 24.5, 25.3, 25.5, 28.4, 35.4, 64.5, 65.5, 126.3, 127.2, 127.9, 136.3; MS (E.I., 70 eV) *m*/*z* 188 ($M^+$).

**2-Methyl-2,3-diphenyloxiran:** $^1H$ NMR (400.1 MHz, $CDCl_3$): δ 1.48 (s, 3 H), 3.98 (s, 1H), 7.30-7.34 (m, 2 H), 7.37-7.42 (m, 6 H), 7.45-7.48 (m, 2 H); $^{13}C$ NMR (100.6 MHz, $CDCl_3$): δ 16.7, 63.0, 67.1, 125.1, 126.5, 127.5, 127.6, 128.2, 128.4, 135.9, 142.3; MS (E.I., 70 eV): *m*/*z* 210 ($M^+$).

**2-Methylindenoxid:** $^1H$ NMR (400.1 MHz, $CDCl_3$): δ 1.69 (s, 3 H), 2.90 (d, *J* = 17.7 Hz, 1 H), 3.15 (d, *J* = 17.7 Hz, 1 H), 4.04 (d, *J* = 1.2 Hz, 1 H), 7.14-7.25 (m, 3 H), 7.44 (d, *J* = 7.3 Hz, 1 H); $^{13}C$ NMR (100.6 MHz, $CDCl_3$): δ 18.5, 38.6, 65.0, 65.3, 124.8, 125.7, 126.0, 128.2, 141.7, 144.5; MS (E.I., 70 eV) *m*/*z* 146 ($M^+$).

**2,2,3-Trimethyl-3-phenyloxiran:** $^1H$ NMR (400.1 MHz, $CDCl_3$): δ 0.95 (s, 3 H), 1.46 (s, 3 H), 1.61 (s, 3 H), 7.20-7.23 (m, 1 H), 7.27-7.33 (m, 4 H); $^{13}C$ NMR (100.6 MHz, $CDCl_3$): δ 20.7, 21.3, 21.7, 63.7, 66.5, 126.0, 126.7, 128.0, 142.2; MS (E.I., 70 eV) *m*/*z* 162 ($M^+$).

***Trans*-2-Hydroxymethyl-3-phenyloxiran:** $^1H$ NMR (400.1 MHz, $CD_2Cl_2$): δ. 1.84 (br s, 1 H), 3.20 (d, *J* = 4.2,2.2 Hz, 1. H), 3.74 (dd, *J* = 12.7, 4.2 Hz, 1 H), 3.88 (d, *J* = 2.2 Hz, 1 H), 4.01 (dd, *J* = 12.7, 2.2 Hz, 1 H), 7.27 - 7.38 (m, 5 H); $^{13}C$ NMR (100.6 MHz, $CD_2Cl_2$): δ 55.8, 61.7, 62.8, 116.7, 126.1, 128.5, 128.8; MS (E.I., 70 eV): *m*/*z* 150 ($[M]^+$, 5), 132 (19), 131 (12), 119 (19), 107 (100), 105 (33), 104 (34), 91 (67), 90 (78), 89 (58), 79 (67), 77 (41).

***Trans*-2-[(*tert*-Butyldimethylsiloxy)methyl]-3-phenyloxiran:** $^1H$ NMR (400.1 MHz, $CDCl_3$): δ 0.09 (s, 3 H), 0.10 (s, 3 H), 0.91 (s, 9 H), 3.12-3.13 (ddd, *J* = 4.4, 2.8, 1.9 Hz, 1 H), 3.79 (d, *J* = 1.9 Hz, 1 H), 3.81 (dd, J = 12.0, 4.4 Hz, 1 H), 3.95 (dd, J = 12.0, 2.8 Hz, 1 H), 7.24-7.35 (m, 5 H); $^{13}C$ NMR (100.6 MHz, $CDCl_3$): δ -5.3, 18.4, 25.9, 55.9, 62.7, 64.0, 125.7, 128.1, 128.4, 137.2; MS (E.I., 70 eV): *m*/*z* 249 ($M^+$- $CH_3$).

**3-Phenyloxiranylmethyl-acetat:** $^1H$ NMR (400.1 MHz, $CDCl_3$): δ 2.04 (s, 3 H), 3.18 - 3.20 (m, 1 H), 3.73 (d, *J* = 2.0 Hz, 1 H), 4.02 (dd, *J* = 12.3, 6.0 Hz, 1 H), 4.41 (dd, *J* = 12.3, 3.4 Hz, 1 H), 7.17 -7.32 (m, 5 H), $^{13}C$ NMR (100.6 MHz, $CDCl_3$): δ 20.7, 56.4, 59.2, 64.2, 125.6, 128.4, 128.5, 136.1, 170.7; MS (E.I., 70 eV): *m*/*z* 192 ($M^+$, 2), 150 (10), 149 (79), 133 (26), 107 (95), 105 (67), 91 (54), 90 (45), 89 (42), 79 (31), 77 (31), 43 (100).

***Trans*-2-Methoxymethyl-3-phenyloxiran:** $^1H$ NMR (400.1 MHz, $CDCl_3$): δ 3.19 (ddd, *J* = 5.2, 3.1, 2.1 Hz, 1 H), 3.43 (s, 3 H), 3.52 (dd, *J* = 11.4, 5.2 Hz, 1 H), 3.76 (dd, *J* = 11.4,3.1 Hz, 1 H), 3.78 (d, *J* = 2.1 Hz, 1 H), 7.25-7.35 (m, 5 H); $^{13}C$ NMR (100.6 MHz, $CDCl_3$): δ 55.7, 59.2, 60.9, 72.1, 125.6, 128.2, 128.4, 136.8; MS (E.I., 70 eV) *m*/*z* 164 ($M^+$).

***Trans*-2-(p-methoxyphenyl)-3-methyloxiran:** $^1H$ NMR (400.1 MHz, $CD_2Cl_2$): δ 1.41 (d, *J* = 5.2 Hz, 3 H), 3.01 (qd, *J* = 5.2, 2.0 Hz, 1 H), 3.50 (d, *J* = 2.0 Hz, 1 H), 3.79 (s, 3 H), 6.87 (d, *J* = 8.9 Hz, 2 H), 7.17 (d, *J* = 8.9 Hz, 2 H); $^{13}C$ NMR (100.6 MHz, $CD_2Cl_2$): δ 18.0, 58.9, 59.5, 114.1, 127.2, 130.3, 160.0; MS (E.I., 70 eV): *m*/*z* = 165 ($[M+1]^+$, 7), 164 ($M^+$, 57), 121 (47), 120 (82), 105 (31), 91 (100), 77 (55), 51 (37).

**Trans-2-Phenoxymethyl-3-phenyl-oxiran:** $^1H$ NMR (400.1 MHz, $CDCl_3$): δ 3.40 (ddd, *J* = 5.2, 3.2,2.0 Hz, 1 H), 3.91, (d, *J* = 2.0 Hz, 1 H), 4.14 (dd, *J* = 11.2, 5.2 Hz, 1 H), 4.32 (dd, *J* = 11.2, 3.2 Hz, 1 H), 6.94-7.00 (m, 3 H), 7.27-7.38 (m, 7 H); $^{13}C$ NMR (100.6 MHz, $CDCl_3$): δ 56.4, 60.2, 67.8, 114.7, 121.3, 125.7, 128.4, 128.5, 129.5, 136.5,

158.4; MS (E.I., 70 eV) *m/z* 226 ($M^+$).

**2-(3-Phenyl-oxiranyl)-[1,3]dioxolan:** $^1H$ NMR (400.1 MHz, $CDCl_3$): δ 3.13 (dd, *J* = 3.8, 2.0 Hz, 1 H), 3.89 (d, *J* = 2.0 Hz, 1 H), 3.89-3.97 (m, 2H), 4.00-4.06 (m, 2 H), 5.00 (d, *J* = 3.8, 1 H), 7.25-7.35 (m, 5 H); $^{13}C$ NMR (100.6 MHz, $CDCl_3$): δ 55.2, 61.3, 65.3, 65.5, 102.3, 125.7, 128.3, 128.4, 136.2; GC-MS: *m/z* 192 ($M^+$).

***Trans*-2-Chlormethyl-3-phenyl-oxiran:** $^1H$ NMR (400.1 MHz, $CDCl_3$): δ 3.28 (ddd, *J* = 5.8, 4.8, 1.9 Hz, 1 H), 3.66 (dd, *J* = 11.8, 5.8 Hz, 1 H), 3.72 (dd, *J* = 11.8, 4.8, Hz, 1 H), 3.82 (d, *J* = 1.9 Hz, 1 H), 7.26-7.38 (m, 5 H); $^{13}C$ NMR (100.6 MHz, $CDCl_3$): δ 44.3, 58.5, 60.9, 116.6, 125.6, 128.6, 135.9; GC-MS: *m/z* 168 ($M^+$).

**2-(3-Phenyloxiranylmethyl)isoindol-1,3-dion:** $^1H$ NMR (400.1 MHz, $CDCl_3$): δ 3.20 (ddd, *J* = 5.7, 4.7, 1.9 Hz, 1 H), 3.82 (dd, *J* = 14.3, 5.7 Hz, 1 H), 3.83 (d, *J* = 1.9 Hz, 1 H), 4.09 (dd, *J* = 14.3, 4.7 Hz, 1 H), 7.19-7.29 (m, 5 H), 7.68 (dd, *J* = 5.5, 3.1 Hz, 2 H),), 7.82 (dd, *J* = 5.5, 3.1 Hz, 2 H); $^{13}C$ NMR (100.6 MHz, $CDCl_3$): δ 39.3, 58.0, 58.9, 123.5, 125.6, 128.4, 128.5, 131.9, 134.2, 136.3, 168.0; MS (E.I., 70 eV): *m/z* 279 ($M^+$).

**Beispiel 26:**

**Synthese des Rutheniumkomplexes (VI-2) [Ru(tpy-β-pinen)(pydic)]**

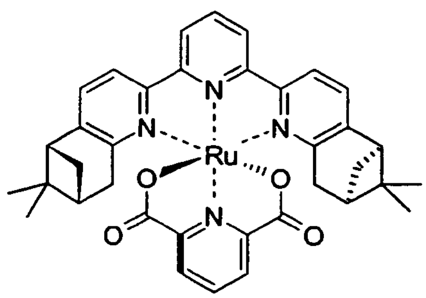

(VI-2)

Es wurden 200 mg (0.47 mmol) des Liganden tpy-β-pinen sowie 145 mg des Rutheniumprecurserkomplexes $[Ru(p\text{-cymol})Cl_2]_2$ (0.24 mmol) in 8 ml Methanol bei Raumtemperatur vorgelegt und eine Lösung von 100 mg das Dinatriumsalzes der Pyridin-2,6-dicarbonsäure ($H_2$pydic) (0.47 mmol), gelöst in 9 ml MeOH/$H_2O$ (2/1) zudosiert. Die Reaktionsmischung wurde 1 h lang auf 65 ˚C erhitzt. Durch Abkühlen wurde das Produkt kristallisiert und man erhielt [Ru(tpy-β-pinen)(pydic)] (61 mg, 19%) als violetten, kristallinen Feststoff.

$R_f$ = 0.20 ($CH_2Cl_2$/MetOH 100:5). $^1H$ NMR (400.1 MHz, $CD_2Cl_2$, ppm) δ 0.44 (s, 6 H), 1.00 (d, *J* = 9.9 Hz, 2 H), 1.26 (s, 6 H), 1.88-1.92 (m, 2 H), 2.30-2.31 (m, 4 H), 2.43-2.48 (m, 2 H), 2.69-2.71 (m, 2 H), 7.27 (d, *J* = 7.9 Hz, 2 H), 7.75 (t, *J* = 8.1 Hz, 1 H), 8.00 (d, *J* = 7.9 Hz, 2 H), 8.08 (t, *J* = 7.7 Hz, 1 H), 8.24 (d, , *J* = 8.1 Hz, 2 H), 8.30 (d, , *J* = 7.7 Hz, 2 H). $^{13}C$ NMR (100.6 MHz, $CD_2Cl_2$, ppm) δ 20.6, 24.9, 30.2, 34.2, 38.3, 40.0, 47.1, 119.3, 120.3, 127.6, 130.5, 133.3, 133.8, 145.7, 155.0, 157.8, 158.2, 164.3, 172.7. FAB-MS *m/z* 688 ($M^+$). UV-VIS ($CH_2Cl_2$, $\lambda_{max}$/nm, log ε) 339 (4.57), 399 (3.95), 524 (3.94) 569 (sh, 3.90). Elementaranalyse ber. für $C_{36}H_{34}N_4O_4Ru\cdot H_2O$ (%) C 61.27, H 5.14, N 7.94; gef. C 61.65, H 5.18, N 7.80.

**Beispiel 27:**

**Synthese des Rutheniumkomplexes (VI-3) [Ru(tpy-myrt)(pydic)]:**

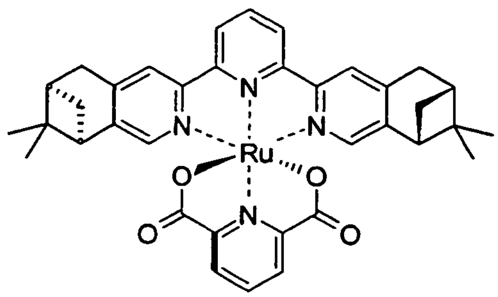

(VI-3)

Die Synthese erfolgt wie unter Beispiel 26 beschrieben unter Verwendung von 211 mg Liganden tpy-myrt (0.50 mmol), 105 mg und $Na_2$pydic (0.50 mmol) sowie 152 mg $[Ru(p\text{-cymol})Cl_2]_2$ (0.25 mmol). Es wurden300 mg Ru(tpy-myrt)(pydic) (VI-3) (90%) als violetter, kristalliner Feststoff erhalten.

$R_f$ = 0.28 ($CH_2Cl_2$/MeOH 100:5). $^1$H NMR (400.1 MHz, $CD_2Cl_2$): δ 0.62 (s, 6 H), 1.14-1.17 (m, 2 H), 1.35 (s, 6 H), 2.28-2.32 (m, 2 H), 2.62-2.64 (m, 4 H), 3.24-3.26 (m, 4 H), 7.09 (s, 2 H), 7.76 (t, *J* = 8.0 Hz, 1 H), 8.05 (s, 2 H), 8.09 (t, *J* = 7.8 Hz, 1 H), 8.25 (d, *J* = 8.0 Hz, 2 H), 8.31 (d, *J* = 7.8 Hz, 2 H); $^{13}$C NMR (100.6 MHz, $CD_2Cl_2$): δ 21.1, 25.5, 31.3, 32.8, 38.9, 39.8, 44.9, 119.6, 121.5, 127.3, 129.2, 133.4, 145.0, 146.5, 146.7, 151.1, 157.6, 157.7, 172.1; FAB-MS (E.I., 70 eV) *m/z* 688 ($M^+$); UV-VIS ($CH_2Cl_2$, $\lambda_{max}$/nm, log ε) 332 (4.58), 392 (4.08), 518 (4.01). Elementaranalyse ber. für $C_{36}H_{34}N_4O_4Ru \cdot 0.5CH_2Cl_2$ (%) C 60.04, H 4.83, N 7.67; gef. C 59.91, H 5.08, N 7.86.

**<u>Beispiel 28:</u>**

**Synthese des Rutheniumkomplexes (VI-4) [Ru(tpy-Me$_2$-β-pinen)(pydic)]:**

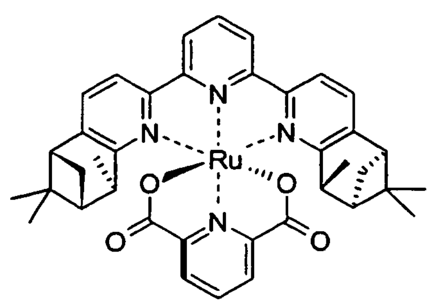

(VI-4)

tpy-$Me_2$-β-pinen (50 mg, 0.11 mmol) und $RuCl_3 \cdot xH_2O$ (29 mg, 0.11 mmol) wurden bei 125 °C in *n*-Butanol über Nacht gerührt. Danach wurden Pyridin-2,6-dicarbonsäure (19 mg, 0.11 mmol) und Triethylamin (46.6 μL, 0.33 mmol) zugefügt und die Reaktionsmischung für eine weitere Stunde bei 125 °C gerührt. Nach Entfernen des Solvens bei vermindertem Druck wurde das Produkt chromatographisch gereinigt (Kieselgel mit 100:2 bis 100:5 $CH_2Cl_2$:Methanol als Gradienten-Eluent) und 41 mg (VI-4) als violetten, kristallinen Feststoff erhalten (57%). Analysenreine Substanz wurde durch Umkristallisation aus $CH_2Cl_2$/*n*-Hexan erhalten.

$R_f$ = 0.12 ($CH_2Cl_2$/MeOH 100:5). $^1$H NMR (400.1 MHz, $CD_2Cl_2$, ppm) δ 0.54 (s, 6 H), 0.60 (d, *J* = 6.9 Hz, 6 H), 1.17 (d, *J* = 10.3 Hz, 2 H), 1.32 (s, 6 H), 1.73 (m, 2 H), 1.94 (ddd, *J* = 13.8, 6.9, 3.6 Hz, 2 H), 2.35 (m, 2 H), 2.70 (m, 2 H), 7.23 (d, *J* = 7.8 Hz, 2 H), 7.81 (t, *J* = 8.1 Hz, 1 H), 7.92 (d, *J* = 7.8 Hz, 2 H), 8.09 (t, *J* = 7.7 Hz, 1 H), 8.18 (d, *J* = 8.1 Hz, 2 H), 8.31 (d, *J* = 7.7 Hz, 2 H). $^{13}$C NMR (100.6 MHz, $CD_2Cl_2$, ppm) δ 20.9, 21.1, 25.2, 27.3, 37.9, 40.5, 47.0, 48.1, 119.2, 120.7, 127.2, 131.6, 133.7, 134.1, 144.5, 155.7, 158.6, 159.1, 169.7, 173.2. FAB-MS *m/z* 716 ($M^+$). UV-VIS ($CH_2Cl_2$, $\lambda_{max/nm,}$ log ε) 338 (4.54), 396 (3.94), 522 (3.89). HRMS ber. für ($C_{38}H_{38}N_4O_4{}^{102}Ru + H^+$) *m/z* 758.22803 gef.

758.22870.

Die Liganden tpy-β-pinen, tpy-myrt und tpy-$Me_2$-β-pinen

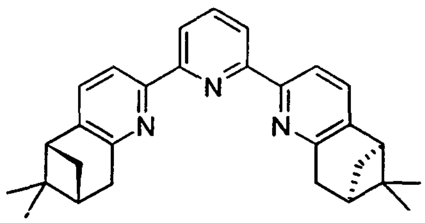

tpy-*β*-pinen

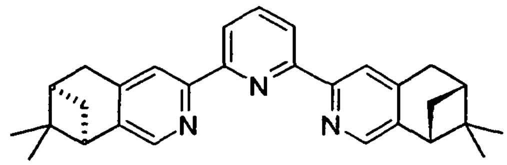

tpy-myrt

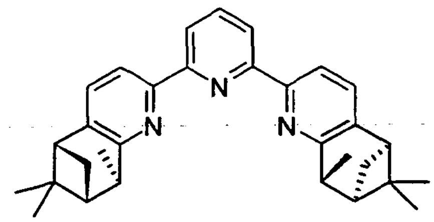

tpy-$Me_2$-*β*-pinen

können beispielsweise gemäß Ziegler, M.; Monney, V.; Stoeckli-Evans, H.; Von Zelewsky, A.; Sasaki, I.; Dupic, G.; Daran, J. C.; Balavoine, G. G. A. Dalton Trans. 1999, 667-675 oder Kwong, H.-L.; Lee, W.-S. Tetrahedron: Asymmetry 2000, 11, 2299-2308 hergestellt werden.

**Beispiel 29:**

**Synthese des Rutheniumkomplexes (VI-5) [Ru(tpy-cam)(pydic)]:**

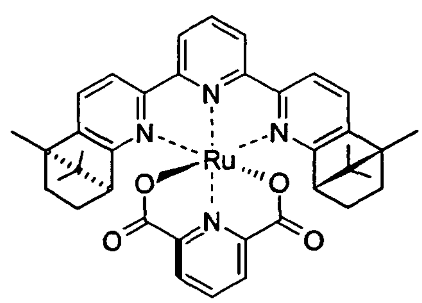

(VI-5)

tpy-cam (137 mg, Rohprodukt, 0.30 mmol) (Herstellung vgl. Beispiel 30) und $RuCl_3xH_2O$ (80 mg, 0.30 mmol) wurden über Nacht bei 125 °C in *n*-Butanol gerührt. Pyridin-2,6-dicarbonsäure (61 mg, 0.30 mmol) und $Et_3N$ (128 µL, 0.91 mmol) wurden dann zugefügt und die Reaktionsmischung wurde für eine weitere Stunden gerührt. Nach Entfernen des Solvens bei vermindertem Druck wurde das Produkt chromatographisch gereinigt (Kieselgel mit 100:0 bis 100:5 $CH_2Cl_2$:Methanol als Gradienten-Eluent) und 18 mg (VI-5) als violetter, kristalliner Feststoff erhalten (18 mg, 8 %). Analysenreine Substanz wurde durch Umkristallisation aus $CH_2Cl_2$/*n*-Hexan erhalten.

$R_f$ = 0.18 ($CH_2Cl_2$/MeOH 100:5). $^1H$ NMR (400.1 MHz, $CD_2Cl_2$, ppm) δ 0.35 (s, 6 H), 0.73 (s, 6 H), 0.83-0.87 (m, 2 H), 1.01-1.07 (m, 2 H), 1.21 (s, 6 H), 1.39 (d, *J* = 3.89 Hz, 2 H), 1.71-1.77 (m, 4 H), 7.38 (d, *J* = 7.5 Hz, 2 H), 7.73 (t, *J* = 7.2 Hz, 1 H), 8.07 (d, *J* = 7.7 Hz, 2 H), 8.12 (t, *J* = 7.7 Hz, 1 H), 8.22 (d, *J* = 7.5 Hz, 2 H), 8.34 (d, , *J* = 7.7 Hz, 2

H). $^{13}C$ NMR (100.6 MHz, $CD_2Cl_2$, ppm) δ 10.7, 18.1, 19.4, 25.6, 32.3, 52.1, 53.4, 56.3, 119.7, 120.0, 126.5, 127.6, 130.5, 133.1, 148.9, 153.8, 156.3, 157.8, 172.5, 174.3. FAB-MS *m/z* 717 (M + $H^+$). UV-VIS ($CH_2Cl_2$, $\lambda_{max}$/nm, log ε) 335 (4.49), 386 (3.90), 519 (3.85). HRMS ber. für ($C_{38}H_{38}N_4O_4{}^{102}Ru$ + $H^+$) *m/z* 717.20148 gef. 717.20068.

**Beispiel 30:**

**Synthese des Liganden tpy-cam:**

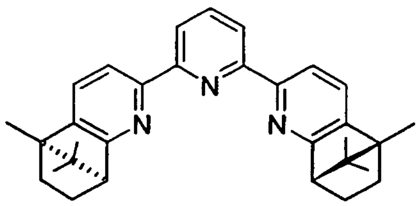

tpy-cam

**a) Herstellung von 2-Methylenbornan** (vgl. Greenwald, R.; Chaykovsky, E. J.; Corey, E. J. *J. Org. Chem.* **1962**, *28*, 1128-1129.):

Zu einer Lösung von Natriumhydrid (1.2 g, 0.06 mmol) in DMSO (20 g) wurden bei Raumtemperatur Methyltriphenylphosphoniumbromid (17.9 g, 0.05 mmol), gelöst in DMSO (50 mL), und Campher (6 g, 0.04 mmol), gelöst in DMSO (20 mL), gegeben. Diese Mischung wurde dann für 72 h bei 60 °C gerührt. Nach Hydrolyse (50 g Wasser) und Extraktion mit *n*-Pentan wurde das organische Solvens entfernt und der Rückstand chromatographiert (Kieselgel, *n*-Pentan) und farblose Kristalle von 2-Methylenbornan (2.12 g, 36 %) erhalten.

Schmelzpunkt: 65 - 67 °C; $^1H$ NMR (400.1 MHz, $CD_2Cl_2$): δ 0.75 (s, 3 H), 0.89 (s, 3 H), 0.91 (s, 3 H), 1.14-1.27 (m, 2 H), 1.60-1.68 (m, 1 H), 1.70-1.83 (m, 2 H), 1.87-1.94 (m, 1H), 2.35-2.43 (m, 1 H), 4.61-4.70 (m, 2 H); $^{13}C$ NMR (100.6 MHz, $CD_2Cl_2$): δ 12.6, 19.0, 19.7, 28.3, 35.5, 37.3, 45.1, 47.5, 51.8, 101.1, 160.1; $[\alpha]_D$ = -41.75° ($CH_2Cl_2$, c = 0.92); MS (E.I., 70 eV): *m/z* 151 ($[M+1]^+$, 3), 150 ($[M]^+$, 25), 135 (54), 121 (28), 108 (21), 107 (100), 95 (37), 94 (52), 93 (61), 91 (48), 79 (68); HRMS ber. für $C_{11}H_{18}$ *m/z* 150.14085 gef. 150.13664.

**b) Herstellung von 2-Methylen-3-oxobornan** (vgl. Hartshorn, M. P.; Wallis, A. F. A. *J. Chem. Soc.* **1964**, 5254-5260):

2-Methylenbornan (1.89 g, 12.6 mmol) und Selendioxid (1.4 g, 12.6 mmol) in Kohlenstofftetrachlorid (5 mL) wurden 14 h am Rückfluss erhitzt. Von der Reaktionsmischung wurde das Solvens entfernt und der Rückstand chromatographiert (Kieselgel, *n*-Hexan), so dass man hellgelbe Kristalle 2-Methylen-3-oxobornan erhielt (873 mg, 42%).

Schmelzpunkt: 69 - 75 °C; *Rf* = 0.4 (*n*-Hexan, Kieselgel); $^1H$ NMR (400.1 MHz, $CD_2Cl_2$): δ 0.82 (s, 3 H), 0.96 (s, 3 H), 1.09 (s, 3 H), 1.37-1.46 (m, 2 H), 1.86 (dd, *J* = 10.5 Hz, 1 H), 1.99 (ddd, *J* = 10.5, 5.2, 2.2 Hz, 1H), 2.20 (d, *J* = 5.2 Hz, 1 H), 5.01 (s, 1 H), 5.74 (d, *J* = 0.6 Hz, 1 H); $^{13}C$ NMR (100.6 MHz, $CD_2Cl_2$): δ 11.9, 17.3, 20.4, 22.7, 34.5, 45.7, 51.6, 59.3, 110.4, 154.7, 255.7; $[\alpha]_D$ =-127.6° ($CH_2Cl_2$, c = 0.64); MS (E.I., 70 eV): *m/z* 165 ($[M+1]^+$, 9), 164 ($[M]^+$, 63), 149 (56), 136 (21), 122 (28), 121 (100), 107 (33), 96 (65), 95 (48), 93 (66), 91 (34), 79 (38), 77 (32), 69 (35), 67 (82), 55 (32), 41 (93), 39 (49), 27 (40). HRMS ber. für ($C_{11}H_{16}O$) *m/z* 164.12012 gef. 164.12049.

**c) Herstellung von tpy-cam:**

2-Methylen-3-oxobornan (773 mg, 4.71 mmol), Ammoniumacetat (726 mg, 9.42 mmol) und 2,6-Bis(pyridinoacetyl)pyridin-diiodid (1.347 g, 2.35 mmol) (hergestellt gemäß Ziegler, M.; Monney, V.; Stoeckli-Evans, H.; Von Zelewsky, A.; Sasaki, I.; Dupic, G.; Daran, J. C.; Balavoine, G. G. A. Dalton Trans. 1999, 667-675 wurden in Essigsäure (10 mL) suspendiert und in einem Druckrohr 14 Stunden lang bei 120 °C gerührt. Nach Neutralisation der Reaktionmischung durch 16 %ige Sodalösung und Extraktion mit Chloroform wurde nach Entfernen des organischen Solvens der Ligand tpy-cam (137 mg, Rohprodukt) erhalten.

**Beispiel 31:**

**Asymmetrische Epoxidierung von 1-Phenyl-2-methylpropen mit Komplex (VI-3) als Katalysator**

Der Rutheniumkomplex (VI-3) (0.0025 mmol) wurde in tert-Amylalkohol (9 ml) bei Raumtemperatur gerührt und 1-Phenyl-2-methylpropen (0.5 mmol) zugesetzt. Zu dieser Mischung wurde eine Lösung von 30 %igem Wasserstoffperoxid (1.5 mmol) in t-Amylalkohol (0.83 ml) innerhalb von 12 h dosiert. Danach wurde die Reaktion durch Zusatz von Wasser (10 ml) und $Na_2SO_3$ (0.5 g) gequencht und mit Ethylacetat (20 ml) extrahiert. Nach Trocknung der organischen Phase wurde die Epoxid-Ausbeute mittels Gaschromatographie bestimmt. Ausbeute: 96 % d. Th.; Enantiomerenüberschuß = +54 % ((R)-(+)-1-Phenyl-2-methylpropen-oxid ist das überschüssige Enantiomer).

Der Enantiomerenüberschuß wurde mittels HPLC an einem chiralen Säulenmaterial bestimmt und über den nachfolgend definierten ee ("enantiomeric excess") (S) oder (R)-Wert angegeben.

Der ee-Wert ergibt sich dabei über folgende Formeln:

$$ee\,(S) = \frac{m(S)-m(R)}{m\,(S+R)} \times 100\%$$

$$ee\,(R) = \frac{m(R)-m(S)}{m\,(S+R)} \times 100\%$$

wobei ee (S) bzw. ee (R) die optische Reinheit des Enantiomers S bzw. R ist, m(S) die Stoffmenge des Enantiomers S und m(R) die Stoffmenge des Enantiomers R ist. (Beispiele: Für ein Racemat gilt: R=S => ee = 0; für die reine (S)-Form gilt: ee (S) = 100 %; für ein Verhältnis von S:R = 9:1 ist ee (S) = 80 %)

**Patentansprüche**

1. Verfahren zur Herstellung von Verbindungen der Formel (I),

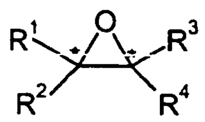

(I)

in der

$R^1$, $R^2$, $R^3$ und $R^4$ jeweils unabhängig voneinander für Wasserstoff, Alkyl, Aryl, Arylalkyl, Halogenalkyl oder Reste der Formel (IIa) bis (IIf) stehen

| | | | |
|---|---|---|---|
| A-B-D-E | (IIa) | A-E | (IIb) |
| $A\text{-}SO_2\text{-}E$ | (IIc) | $A\text{-}B\text{-}SO_2R^6$ | (IId) |
| $A\text{-}SO_3W$ | (IIe) | A-COW | (IIf) |

wobei in den Formeln (IIa) bis (IIf)

A fehlt oder für einen Alkylen- oder Halogenalkylenrest steht und
B fehlt oder für Sauerstoff oder $NR^5$ steht, wobei
$R^5$ für Wasserstoff, Arylalkyl oder Aryl steht und

D für eine Carbonyl-Gruppe steht und
E für $R^6$, $OR^6$, $NHR^7$ oder $N(R^7)_2$ steht,

wobei

$R^6$ für Alkyl, Arylalkyl oder Aryl und
$R^7$ jeweils unabhängig für Alkyl, Arylalkyl oder Aryl steht oder $N(R^7)_2$ zusammen für einen cyclischen Aminorest mit 4 bis 12 Kohlenstoffatomen steht und
W für OH, $NH_2$, oder OM steht, wobei M ein Alkalimetallion, ein halbes Äquivalent eines Erdalkalimetallions, ein Ammoniumion oder ein organisches Ammoniumion steht

oder jeweils zwei der Reste $R^1$, $R^2$, $R^3$ und $R^4$ zusammen Teil eines 3 bis 7-gliedrigen Cyclus sind, der insgesamt 3 bis 16 Kohlenstoffatome umfasst,
das **dadurch gekennzeichnet ist, dass** Verbindungen der Formel (III),

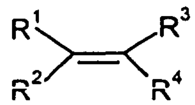

(III)

in der $R^1$, $R^2$, $R^3$ und $R^4$ jeweils unabhängig voneinander die vorstehend genannte Bedeutung besitzen,
mit Wasserstoffperoxid umgesetzt werden,
wobei die Umsetzung in Gegenwart eines Rutheniumkomplexes erfolgt, der als Liganden **sowohl** Verbindungen der Formel (IV) trägt

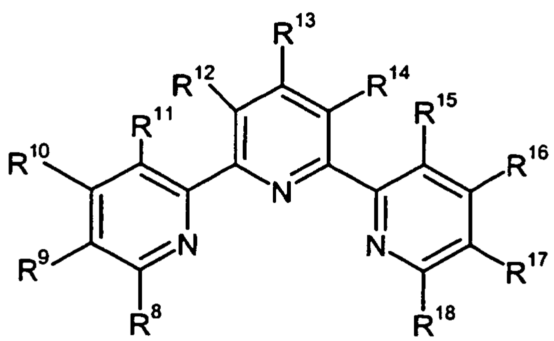

(IV)

in der $R^8$, $R^9$, $R^{10}$, $R^{11}$, $R^{12}$, $R^{13}$, $R^{14}$, $R^{15}$, $R^{16}$, $R^{17}$ und $R^{18}$ jeweils unabhängig voneinander für Wasserstoff, Halogen, Hydroxy, Hydroxycarbonyl, Alkoxycarbonyl, Alkoxy, Alkyl, Arylalkyl oder Aryl stehen, oder
jeweils zwei der Reste $R^8$, $R^9$, $R^{10}$ und $R^{11}$ oder jeweils zwei der Reste $R^{15}$, $R^{16}$, $R^{17}$ und $R^{18}$ zusammen Teil eines 3 bis 7-gliedrigen Monocyclus sind, der insgesamt 3 bis 16 Kohlenstoffatome umfasst, oder zusammen Teil eines Bicyclus sind, der insgesamt 3 bis 16 Kohlenstoffatome umfasst,
als auch Verbindungen der Formel (V) trägt

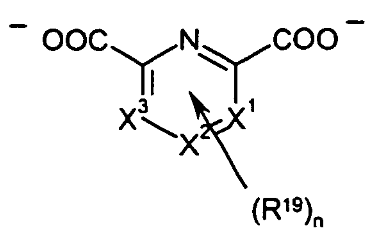

(V)

in der

$X^1$, $X^2$ und $X^3$ jeweils unabhängig voneinander für N, CH bzw. $CR^{19}$ stehen und
$R^{19}$ für Wasserstoff, Halogen, Hydroxy, Hydroxycarbonyl, Alkoxycarbonyl, Alkoxy, Alkoxyalkyl, Arylalkyl oder Aryl steht und
n für 0, 1, 2 oder 3 bevorzugt für 0 oder 1 und besonders bevorzugt für 0 steht.

**2.** Verfahren nach Anspruch 1, **dadurch gekennzeichnet, dass** in Formel (I) $R^1$, $R^2$, $R^3$ und $R^4$ bevorzugt jeweils unabhängig voneinander für Wasserstoff, gegebenenfalls substituiertes $C_1$-$C_8$-Alkyl, gegebenenfalls substituiertes $C_5$-$C_{14}$-Aryl, gegebenenfalls substituiertes $C_6$-$C_{15}$-Arylalkyl oder $C_1$-$C_8$-Halogenalkyl stehen oder jeweils zwei der Reste $R^1$, $R^2$, $R^3$ und $R^4$ zusammen Teil eines 3 bis 7-gliedrigen Cyclus sind, der insgesamt 3 bis 16 Kohlenstoffatome umfasst.

**3.** Verfahren nach Anspruch 1 oder 2, **dadurch gekennzeichnet, dass** in Formel (I) mindestens ein Rest aus $R^1$, $R^2$, $R^3$ und $R^4$ für gegebenenfalls substituiertes $C_5$-$C_{14}$-Aryl steht oder jeweils zwei der Reste $R^1$, $R^2$, $R^3$ und $R^4$ zusammen Teil eines 3 bis 7-gliedrigen Cyclus sind, der insgesamt 3 bis 16 Kohlenstoffatome umfasst.

**4.** Verfahren nach wenigstens einem der Ansprüche 1 bis 3, **dadurch gekennzeichnet, dass** in Formel (IV) Formel $R^8$, $R^9$, $R^{11}$, $R^{12}$, $R^{14}$, $R^{15}$, $R^{17}$ sowie $R^{18}$ für Wasserstoff und gleichzeitig $R^{10}$, $R^{13}$ sowie $R^{16}$ für tert-Butyl stehen, $R^8$, $R^9$, $R^{10}$, $R^{11}$, $R^{12}$, $R^{13}$, $R^{14}$, $R^{15}$, $R^{16}$, $R^{17}$ und $R^{18}$ für Wasserstoff stehen oder jeweils zwei der Reste $R^8$, $R^9$, $R^{10}$ und $R^{11}$ und jeweils zwei der Reste $R^{15}$, $R^{16}$, $R^{17}$ und $R^{18}$ zusammen Teil eines 3 bis 7-gliedrigen Monocyclus sind, der insgesamt 3 bis 16 Kohlenstoffatome umfasst, oder zusammen Teil eines Bicyclus sind, der insgesamt 3 bis 16 Kohlenstoffatome umfasst, und die übrigen Reste aus $R^8$, $R^9$, $R^{10}$, $R^{11}$, $R^{12}$, $R^{13}$ $R^{14}$, $R^{15}$, $R^{16}$, $R^{17}$ und $R^{18}$ für Wasserstoff stehen.

**5.** Verfahren nach wenigstens einem der Ansprüche 1 bis 4, **dadurch gekennzeichnet, dass** in Formel (IV) $R^8$, $R^9$, $R^{10}$, $R^{11}$, $R^{12}$, $R^{13}$, $R^{14}$, $R^{15}$, $R^{16}$, $R^{17}$ und $R^{18}$ für Wasserstoff stehen.

**6.** Verfahren nach wenigstens einem der Ansprüche 1 bis 4, **dadurch gekennzeichnet, dass** in Formel (V) von den Resten $X^1$, $X^2$, $X^3$ mindestens zwei, bevorzugt drei für CH oder $CR^{19}$ stehen.

**7.** Verfahren nach wenigstens einem der Ansprüche 1 bis 6, **dadurch gekennzeichnet, dass** als Rutheniumkomplexe solche der Formel (VI) eingesetzt werden

[Ru(IV)(V)] (VI)

in der (IV) für eine Verbindung der Formel (IV) und (V) für eine Verbindung der Formel (V) steht oder solche die in situ in der Reaktionsmischung aus einem geeigneten Rutheniumprecursor und den beiden Liganden der Formeln (IV) und (V) in der Reaktionsmischung erzeugt werden.

**8.** Verfahren nach wenigstens einem der Ansprüche 1 bis 7, **dadurch gekennzeichnet, dass** es in Gegenwart von sekundären oder tertiären Alkoholen als Lösungsmittel durchgeführt wird.

**9.** Verbindungen der Formeln (VI-2) bis (VI-5)

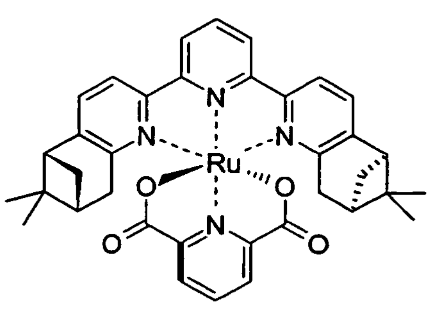

(VI-2)

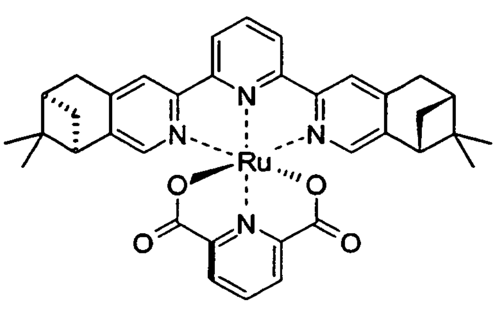

(VI-3)

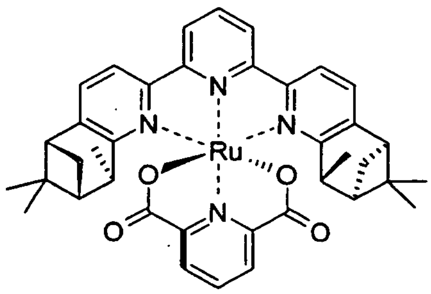

(VI-4)

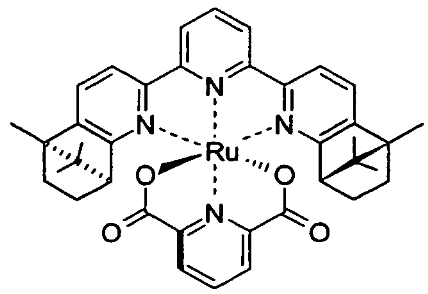

(VI-5)

## Claims

**1.** Process for preparing compounds of the formula (I),

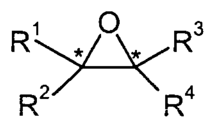

(I)

where

$R^1$, $R^2$, $R^3$ and $R^4$ are each, independently of one another, hydrogen, alkyl, aryl, arylalkyl, haloalkyl or a radical of one of the formulae (IIa) to (IIf)

| | | | |
|---|---|---|---|
| A-B-D-E | (IIa) | A-E | (IIb) |
| A-$SO_2$-E | (IIc) | A-B-$SO_2R^6$ | (IId) |
| A-$SO_3$W | (IIe) | A-COW | (IIf) |

where, in the formulae (IIa) to (IIf)

A is absent or is an alkylene or haloalkylene radical and
B is absent or is oxygen or $NR^5$, where
$R^5$ is hydrogen, arylalkyl or aryl, and
D is a carbonyl group and
E is $R^6$, $OR^6$, $NHR^7$ or $N(R^7)_2$,

where

$R^6$ is alkyl, arylalkyl or aryl and
the radicals $R^7$ are each, independently of one another, alkyl, arylalkyl or aryl or the moiety $N(R^7)_2$ is a cyclic amino radical having from 4 to 12 carbon atoms, and
W is OH, $NH_2$, or OM, where M is an alkali metal ion, half an equivalent of an alkaline earth metal ion, an ammonium ion or an organic ammonium ion,

or two of the radicals $R^1$, $R^2$, $R^3$ and $R^4$ are together part of a 3- to 7-membered ring having a total of from 3 to 16 carbon atoms,

which is **characterized in that** compounds of the formula (III),

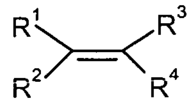

(III)

where $R^1$, $R^2$, $R^3$ and $R^4$ are, in each case independently of one another, as defined above, are reacted with hydrogen peroxide, with the reaction being carried out in the presence of a ruthenium complex which bears as ligands both compounds of the formula (IV)

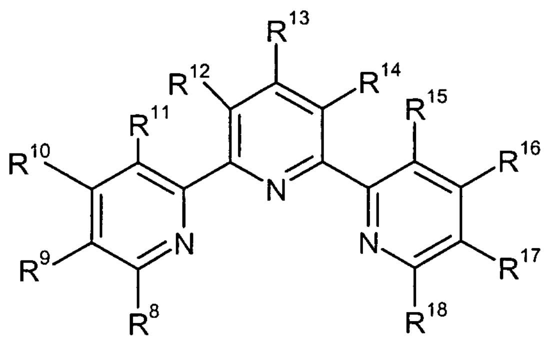

(IV)

where $R^8$, $R^9$, $R^{10}$, $R^{11}$, $R^{12}$, $R^{13}$, $R^{14}$, $R^{15}$, $R^{16}$, $R^{17}$ and $R^{18}$ are each, independently of one another, hydrogen, halogen, hydroxy, hydroxycarbonyl, alkoxycarbonyl, alkoxy, alkyl, arylalkyl or aryl, or two of the radicals $R^8$, $R^9$, $R^{10}$ and $R^{11}$ or two of the radicals $R^{15}$, $R^{16}$, $R^{17}$ and $R^{18}$ are together part of a 3- to 7-membered monocycle having a total of from 3 to 16 carbon atoms or are together part of a bicycle having a total of from 3 to 16 carbon atoms, and also compounds of the formula (V)

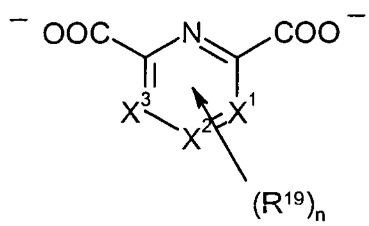

(V)

where

$X^1$, $X^2$ and $X^3$ are each, independently of one another, N, CH or $CR^{19}$ and

$R^{19}$ is hydrogen, halogen, hydroxy, hydroxycarbonyl, alkoxycarbonyl, alkoxy, alkoxyalkyl, arylalkyl or aryl and n is 0, 1, 2 or 3, preferably 0 or 1 and particularly preferably 0.

**2.** Process according to Claim 1, **characterized in that**, in the formula (I), $R^1$, $R^2$, $R^3$ and $R^4$ are each preferably, independently of one another, hydrogen, substituted or unsubstituted $C_1$-$C_8$-alkyl, substituted or unsubstituted $C_5$-$C_{14}$-aryl, substituted or unsubstituted $C_6$-$C_{15}$ -arylalkyl or $C_1$-$C_8$-haloalkyl or two of the radicals $R^1$, $R^2$, $R^3$ and $R^4$ are together part of a 3- to 7-membered ring having a total of from 3 to 16 carbon atoms.

**3.** Process according to Claim 1 or 2, **characterized in that**, in the formula (I), at least one radical $R^1$, $R^2$, $R^3$ and $R^4$ is substituted or unsubstituted $C_5$-$C_{14}$-aryl or two of the radicals $R^1$, $R^2$, $R^3$ and $R^4$ are together part of a 3- to 7-membered ring having a total of from 3 to 16 carbon atoms.

**4.** Process according to at least one of Claims 1 to 3, **characterized in that**, in the formula (IV), $R^8$, $R^9$, $R^{11}$, $R^{12}$, $R^{14}$, $R^{15}$, $R^{17}$ and $R^{18}$ are each hydrogen and at the same time $R^{10}$, $R^{13}$ and $R^{16}$ are each tert-butyl, $R^8$, $R^9$, $R^{10}$, $R^{11}$, $R^{12}$, $R^{13}$, $R^{14}$, $R^{15}$, $R^{16}$, $R^{17}$ and $R^{18}$ are each hydrogen or two of the radicals $R^8$, $R^9$, $R^{10}$ and $R^{11}$ and two of the radicals $R^{15}$, $R^{16}$, $R^{17}$ and $R^{18}$ are together part of a 3- to 7-membered monocycle having a total of from 3 to 16 carbon atoms or are together part of a bicycle having a total of from 3 to 16 carbon atoms and the remaining radicals $R^8$, $R^9$, $R^{10}$, $R^{11}$, $R^{12}$, $R^{13}$, $R^{14}$, $R^{15}$, $R^{16}$, $R^{17}$ and $R^{18}$ are each hydrogen.

**5.** Process according to at least one of Claims 1 to 4, **characterized in that**, in the formula (IV), $R^8$, $R^9$, $R^{10}$, $R^{11}$, $R^{12}$, $R^{13}$, $R^{14}$, $R^{15}$, $R^{16}$, $R^{17}$ and $R^{18}$ are each hydrogen.

**6.** Process according to at least one of Claims 1 to 4, **characterized in that**, in the formula (V), at least two, preferably three, of the radicals $X^1$, $X^2$, $X^3$ are CH or $CR^{19}$,

**7.** Process according to at least one of Claims 1 to 6, **characterized in that** ruthenium complexes used are complexes of the formula (VI)

[Ru(IV)(V)]     (VI)

where (IV) represents a compound of the formula (IV) and (V) represents a compound of the formula (V), or complexes which are generated in situ in the reaction mixture from a suitable ruthenium precursor and the two ligands of the formulae (IV) and (V).

**8.** Process according to at least one of Claims 1 to 7, **characterized in that** it is carried out in the presence of secondary or tertiary alcohols as solvents.

**9.** Compounds of the formulae (VI-2) to (VI-5)

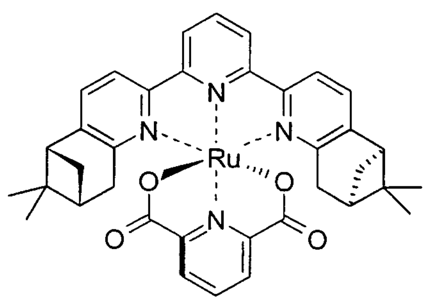

(VI-2)

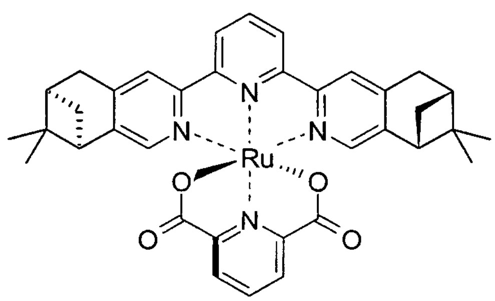

(VI-3)

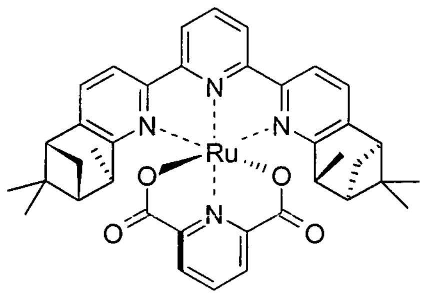

(VI-4)

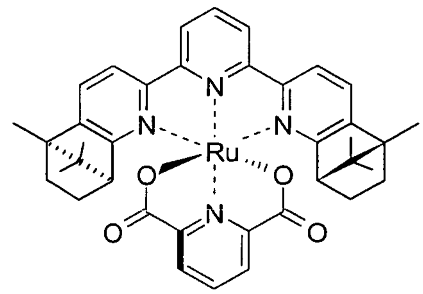

(VI-5)

## Revendications

**1.** Procédé de préparation de composés de la formule (I)

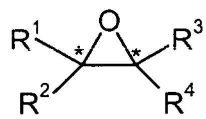

(I)

dans laquelle

$R^1$, $R^2$, $R^3$ et $R^4$ représentent à chaque fois indépendamment les uns des autres de l'hydrogène ou des radicaux alkyle, aryle, arylalkyle, halogénoalkyle ou des restes des formules (IIa) à (IIf),

| | | | |
|---|---|---|---|
| A-B-D-E | (IIa) | A-E | (IIb) |
| A-$SO_2$-E | (IIc) | A-B-$SO_2R^6$ | (IId) |
| A-$SO_3$W | (IIe) | A-COW | (IIf) |

où, dans les formules (IIa) à (IIf) :

A est absent ou représente un reste alkylène ou halogénoalkylène,
B est absent ou représente de l'oxygène ou $NR^5$, avec
$R^5$ étant de l'hydrogène ou un radical arylalkyle ou aryle, et
D représente un groupe carbonyle et
E représente $R^6$, $OR^6$, $NHR^7$ ou $N(R^7)_2$,

où

$R^6$ représente un radical alkyle, arylalkyle ou aryle et
$R^7$ représentent à chaque fois indépendamment les uns des autres des radicaux alkyle, arylalkyle ou aryle, ou bien les $N(R^7)_2$ représentent ensemble un reste amino cyclique comportant de 4 à 12 atomes de carbone, et
W représente OH, $NH_2$ ou OM, où M représente un ion de métal alcalin, un demi-équivalent d'un ion de métal alcalino-terreux, un ion ammonium ou un ion ammonium organique,

ou bien à chaque fois deux des restes $R^1$, $R^2$, $R^3$ et $R^4$ font ensemble partie d'un noyau comportant de 3 à 7 membres, et qui comporte au total de 3 à 16 atomes de carbone, **caractérisé en ce que** l'on fait réagir des composés de la formule (III)

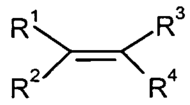

(III)

dans laquelle $R^1$, $R^2$, $R^3$ et $R^4$ possèdent à chaque fois indépendamment les uns des autres la signification indiquée plus haut,
avec du peroxyde d'hydrogène,
la réaction étant entreprise en présence d'un complexe de ruthénium, qui porte comme ligands tant des composés de la formule (IV)

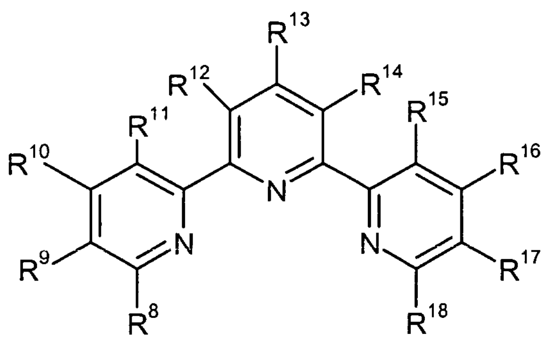

(IV)

dans laquelle $R^8$, $R^9$, $R^{10}$, $R^{11}$, $R^{12}$, $R^{13}$, $R^{14}$, $R^{15}$, $R^{16}$, $R^{17}$ et $R^{18}$ représentent à chaque fois indépendamment les uns des autres de l'hydrogène, un halogène ou des radicaux hydroxy, hydroxycarbonyle, alcoxycarbonyle, alcoxy, alkyle, arylalkyle ou aryle, ou bien
à chaque fois deux des restes $R^8$, $R^9$, $R^{10}$ et $R^{11}$, ou à chaque fois deux des restes $R^{15}$, $R^{16}$, $R^{17}$ et $R^{18}$ font ensemble partie d'un monocycle comportant de 3 à 7 membres, comprenant au total de 3 à 16 atomes de carbone, ou font ensemble partie d'un bicycle comportant au total de 3 à 16 atomes de carbone,
que des composés de la formule (V)

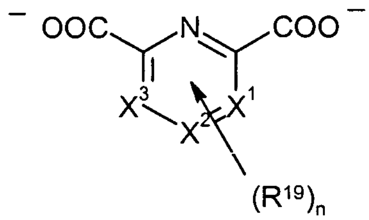

(V)

dans laquelle

$X^1$, $X^2$ et $X^3$ représentent à chaque fois indépendamment les uns des autres N, CH ou $CR^{19}$ et
$R^{19}$ représente de l'hydrogène, un halogène ou un radical hydroxy, hydroxycarbonyle, alcoxycarbonyle, alcoxy,

alcoxyalkyle, arylalkyle ou aryle et
n vaut 0, 1, 2 ou 3, de préférence 0 ou 1 et plus préférablement 0.

**2.** Procédé suivant la revendication 1, **caractérisé en ce que**, dans la formule (I), $R^1$, $R^2$, $R^3$ et $R^4$ représentent de préférence, à chaque fois indépendamment les uns des autres, de l'hydrogène, un radical alkyle en $C_1$ à $C_8$ le cas échéant substitué, un radical aryle en $C_5$ à $C_{14}$ le cas échéant substitué, un radical arylalkyle en $C_6$ à C 15 le cas échéant substitué, ou halogénoalkyle en C $_1$ à $C_8$, ou bien qu'à chaque fois deux des restes $R^1$, $R^2$, $R^3$ et $R^4$ font ensemble partie d'un noyau possédant de 3 à 7 membres et comportant au total de 3 à 16 atomes de carbone.

**3.** Procédé suivant la revendication 1 ou 2, **caractérisé en ce que**, dans la formule (I), au moins un reste parmi $R^1$, $R^2$, $R^3$ et $R^4$ représente un radical aryle en $C_5$ à $C_{14}$ le cas échéant substitué, ou bien qu'à chaque fois deux des restes $R^1$, $R^2$, $R^3$ et $R^4$ font ensemble partie d'un noyau possédant de 3 à 7 membres et comportant au total de 3 à 16 atomes de carbone.

**4.** Procédé suivant au moins l'une quelconque des revendications 1 à 3, **caractérisé en ce que**, dans la formule (IV), $R^8$, $R^9$, $R^{11}$, $R^{12}$, $R^{14}$, $R^{15}$, $R^{17}$ et $R^{18}$ représentent de l'hydrogène et en même temps, $R^{10}$, $R^{13}$ ainsi que $R^{16}$ du tert.-butyle, $R^8$, $R^9$, $R^{10}$, $R^{11}$, $R^{12}$, $R^{13}$, $R^{14}$, $R^{15}$, $R^{16}$, $R^{17}$ et $R^{18}$ représentent de l'hydrogène, ou bien à chaque fois deux des restes $R^8$, $R^9$, $R^{10}$ et $R^{11}$ et à chaque fois deux des restes $R^{15}$, $R^{16}$, $R^{17}$ et $R^{18}$ font ensemble partie d'un monocycle possédant de 3 à 7 membres et comportant au total de 3 à 16 atomes de carbone, ou font ensemble partie d'un bicycle comportant au total de 3 à 16 atomes de carbone, et les autres restes de $R^8$, $R^9$, $R^{10}$, $R^{11}$, $R^{12}$, $R^{13}$, $R^{14}$, $R^{15}$, $R^{16}$, $R^{17}$ et $R^{18}$ représentent de l'hydrogène.

**5.** Procédé suivant au moins l'une quelconque des revendications 1 à 4, **caractérisé en ce que**, dans la formule (IV), $R^8$, $R^9$, $R^{10}$, $R^{11}$, $R^{12}$, $R^{13}$, $R^{14}$, R 15, $R^{16}$, $R^{17}$ et $R^{18}$ représentent de l'hydrogène.

**6.** Procédé suivant au moins l'une quelconque des revendications 1 à 4, **caractérisé en ce que**, dans la formule (V), parmi les restes $X^1$, $X^2$, $X^3$, au moins deux, de préférence trois représentent CH ou $CR^{19}$.

**7.** Procédé suivant au moins l'une quelconque des revendications 1 à 6, **caractérisé en ce que** l'on met en oeuvre en tant que complexes de ruthénium ceux de la formule (VI)

[Ru(IV)(V)] (VI)

dans laquelle (IV) représente un composé de la formule (IV) et (V) un composé de la formule (V), ou ceux qui sont produits in situ dans le mélange réactionnel à partir d'un précurseur de ruthénium approprié et des deux ligands des formules (IV) et (V) dans le mélange réactionnel.

**8.** Procédé suivant au moins l'une quelconque des revendications 1 à 7 **caractérisé en ce qu'**il est entrepris en présence d'alcools secondaires ou tertiaires en tant que solvants.

**9.** Composés des formules (VI-2) à (VI-5)

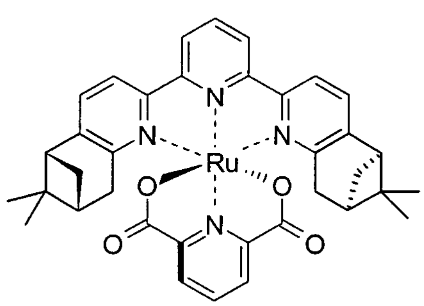

(VI-2)

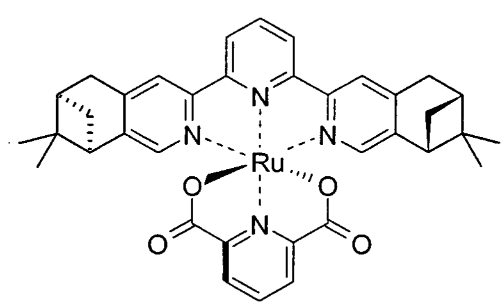

(VI-3)

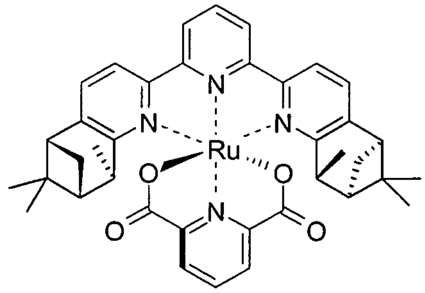

(VI-4)

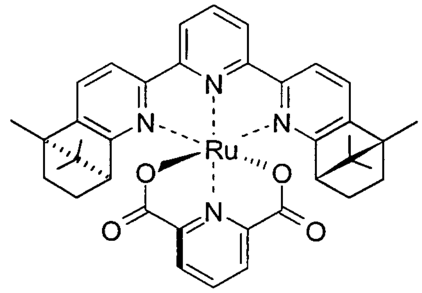

(VI-5)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **HERRMANN, W. A. ; FISCHER, R. W. ; MARZ, D. W.** *Angew. Chem. Int. Ed.,* 1991, vol. 30, 1638-1641
- **RUDOLF, J. ; REDDY, K. L. ; CHIANG, J. P. ; SHARPLESS, K. B.** *J. Am. Chem. Soc.,* 1997, vol. 119, 6189-6190
- **HERMANN, W. A. ; KRATZER, R. M. ; DING, H. ; THIEL, W. R. ; GLAS, H.** *J. Organomet. Chem.,* 1998, vol. 555, 293-295
- **STOOP, R. M. ; BACHMANN, S. ; VALENTINI, M. ; MEZZETTI, A.** *Organornetallics,* 2000, vol. 19, 4117-4126
- **KLAWONN, M. ; TSE, M. K. ; BHOR, S. ; DÖBLER, C. ; BELLER, M.** *J. Mol. Catal. A,* 2004, vol. 218, 13-19
- **NISHIYAMA, H. ; SHIMADA, T. ; ITOH, H. ; SUGIYAMA, H. ; MOTOYAMA, Y.** *Chem. Commun.,* 1997, 1863-1864
- **ZIEGLER, M. ; MONNEY, V. ; STOECKLI-EVANS, H. ; VON ZELEWSKY, A. ; SASAKI, I. ; DUPIC, G. ; DARAN, J. C. ; BALAVOINE, G. G. A.** *Dalton Trans.,* 1999, 667-675
- **KWONG, H.-L. ; LEE, W.-S.** *Tetrahedron: Asymmetry,* 2000, vol. 11, 2299-2308